# EUROPÄISCHE PATENTSCHRIFT

(11) **EP 0 739 876 B1**
(45) Veröffentlichungstag und Bekanntmachung des Hinweises auf die Patenterteilung: **08.03.2000**
(21) Anmeldenummer: 96105868.2
(22) Anmeldetag: 15.04.1996
(51) Int. Cl.: C07C 25/24, C09K 19/30, C07C 43/225, C07C 255/50

(54) **Fluorierte Biphenylcyclohexane und flüssigkristallines Medium**
Fluorinated biphenylcyclohexanes and liquid crystalline medium
Biphénylcyclohexanes fluorés et milieu cristal liquide

(30) Priorität: 27.04.1995 DE 19515504
(43) Veröffentlichungstag der Anmeldung: 30.10.1996
(73) Patentinhaber: MERCK PATENT GmbH, 64293 Darmstadt (DE)
(72) Erfinder: Bremer, Matthias, Dr., 64295 Darmstadt (DE); Tarumi, Kazuaki, Dr., 64342 Seeheim-Jugenheim (DE); Krause, Joachim, Dr., 64807 Dieburg (DE)

(56) Entgegenhaltungen:
- EP-A- 0 475 273
- EP-A- 0 628 532
- EP-A- 0 647 696
- WO-A-91/02709
- WO-A-92/02597
- WO-A-92/13928
- WO-A-92/21734
- WO-A-96/05159
- DE-A- 4 137 401
- DE-A- 4 329 592
- DE-A- 4 414 874
- DE-A- 4 416 256
- DE-A- 4 416 272
- DE-A- 19 513 007
- DE-A- 19 528 085
- GB-A- 2 257 701
- CHEMICAL ABSTRACTS, vol. 123, no. 24, 11.Dezember 1995 Columbus, Ohio, US; abstract no. 325869, OOSAWA M ET AL: "Manufacture of fluorobiphenyl derivatives for liquid crystal materials" XP002034476 & JP 07 179 374 A (DAINIPPON INK & CHEMICALS;JAPAN) 18.Juli 1995

## Beschreibung

Flüssigkristallines Medium enthaltend ein oder mehrere fluorierte Biphenylcyclohexane der Formel I, 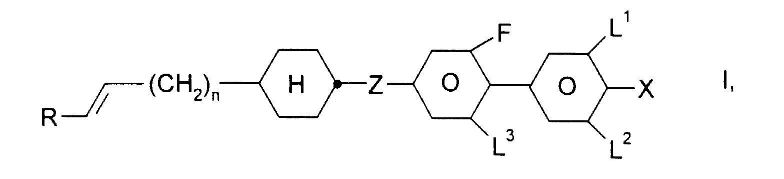 worin
- R: H, Alkyl-, Oxaalkyl- oder Oxaalkenylrest mit 1-6 C-Atomen,
- X: F, Cl, CN, ein durch ein oder mehrere Fluoratome substituierter Alkyl-, Alkoxy-, Alkenyloxy oder Alkenylrest mit 1 bis 6 C-Atomen,
- L^{1,} L² und L³: jeweils unabhängig voneinander H oder F,
- Z: -CH₂CH₂-, -CH=CH-, -C≡C- oder eine Einfachbindung, und
- n: 0, 1, 2 oder 3,
bedeuten,
und ein oder mehrere Verbindungen ausgewählt aus der Gruppe bestehend aus den allgemeinen Formeln II, III und IV, 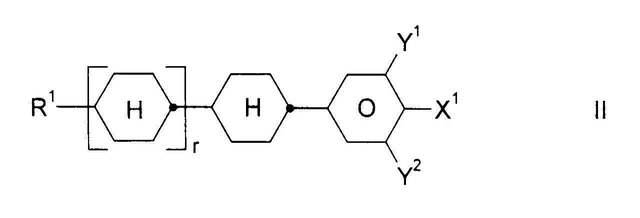 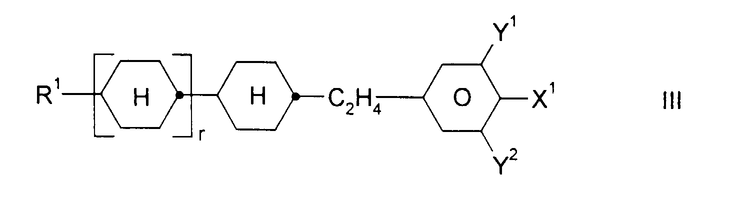 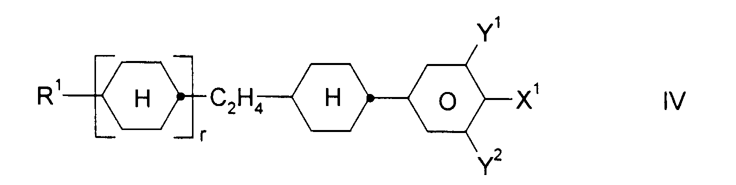 worin
- R¹:: Alkyl, Oxaalkyl, Fluoralkyl oder Alkenyl mit jeweils bis zu 7 C-Atomen
- X¹:: F, Cl, CF₃, OCF₃ oder OCHF₂
- Y¹ und Y²:: jeweils unabhängig voneinander H oder F und
- r:: 0 oder 1
bedeuten,
wobei der Anteil an Verbindungen der Formeln I bis IV im Gesamtgemisch mindestens 50 Gew.-% beträgt.

Die Erfindung betrifft weiterhin Flüssigkristall- und elektrooptische Anzeigeelemente, die die erfindungsgemäßen flüssigkristallinen Medien enthalten.

Die Verbindungen der Formel I können als Komponenten flüssigkristalliner Medien verwendet werden, insbesondere für Displays, die auf dem Prinzip der verdrillten Zelle, dem Guest-Host-Effekt, dem Effekt der Deformation aufgerichteter Phasen oder dem Effekt der dynamischen Streuung beruhen.

Ähnliche Verbindungen werden in der WO 92/13928 A, DE 44 16 256 A, DE 44 14 874 A, EP 0628 532 A, WO 92/21734 A, WO 92/02597 A, DE 42 29 592, GB 2 257 701 A, DE 41 37 401 A, WO 91/02709 und WO 91/08184 beschrieben. Die erfindungsgemäßen Verbindungen werden dort aber nicht genannt.

Die bisher für diesen Zweck eingesetzten Substanzen besitzen sämtlich gewisse Nachteile, beispielsweise zu hohe Schmelzpunkte, zu niedrige Klärpunkte, zu geringe Stabilität gegenüber der Einrichtung von Wärme, Licht oder elektrischen Feldern, zu niedriger elektrischer Widerstand, zu hohe Temperaturabhängigkeit der Schwellenspannung, ungünstige dielektrische und/oder elastische Eigenschaften.

Der Erfindung lag die Aufgabe zugrunde, neue stabile flüssigkristalliner Medien geeignet sind und insbesondere gleichzeitig eine vergleichsweise geringe Viskosität besitzen sowie eine relativ hohe dielektrische Anisotropie.

Es wurde nun gefunden, daß Verbindungen der Formel I als Komponenten flüssigkristalliner Medien vorzüglich geeignet sind. Insbesondere verfügen sie über vergleichsweise niedrige Viskositäten, breite nematische Phasenbereiche und vergleichsweise hohe Klärpunkte. Mit ihrer Hilfe lassen sich stabile flüssigkristalline Medien mit breitem Mesophasenbereich und vorteilhaften Werten für die optische und dielektrische Anisotropie erhalten. Diese Medien weisen ferner ein sehr gutes Tieftemperaturverhalten auf.

Im Hinblick auf die verschiedensten Einsatzbereiche derartiger Verbindungen mit hohem Δε war es jedoch wünschenswert, weitere Verbindungen mit hoher Nematogenität zur Verfügung zu haben, die auf die jeweiligen Anwendungen genau maßgeschneiderte Eigenschaften aufweisen.

Mit der Bereitstellung von Verbindungen der Formel I wird außerdem ganz allgemein die Palette der flüssigkristallinen Substanzen, die sich unter verschiedenen anwendungstechnischen Gesichtspunkten zur Herstellung flüssigkristalliner Gemische eignen, erheblich verbreitert.

Die Verbindungen der Formel I besitzen einen breiten Anwendungsbereich. In Abhängigkeit von der Auswahl der Substituenten können diese Verbindungen als Basismaterialien dienen, aus denen flüssigkristalline Medien zum überwiegenden Teil zusammengesetzt sind; es können aber auch Verbindungen der Formel I flüssigkristallinen Basismaterialien aus anderen Verbindungsklassen zugesetzt werden, um beispielsweise die dielektrische und/oder optische Anisotropie eines solchen Dielektrikums zu beeinflussen und/oder um dessen Schwellenspannung und/oder dessen Viskosität zu optimieren.

Die Verbindungen der Formel I sind in reinem Zustand farblos und bilden flüssigkristalline Mesophasen in einem für die elektrooptische Verwendung günstig gelegenen Temperaturbereich. Chemisch, thermisch und gegen Licht sind sie stabil.

Gegenstand der Erfindung sind somit die Verbindungen der Formel I sowie die Verwendung dieser Verbindungen als Komponenten flüssigkristalliner Medien. Gegenstand der Erfindung sind ferner flüssigkristalline Medien mit einem Gehalt an mindestens einer Verbindung der Formel I sowie Flüssigkristallanzeigeelemente, insbesondere elektroopische Anzeigeelemente, die derartige Medien enthalten.

Bevorzugt sind Verbindungen der Formel I, worin L¹ und/oder L² Fluor bedeuten.

X bedeutet vorzugsweise F, Cl, CN, OCF₃, CF₃, CHF₂, OCHF₂, OCHFCF₃, OCH₂CHF₂, OCH₂CF₃, OCF₂CHF₂, OC₂F₅, OC₃F₇, OCH₂C₂F₅, OCH=CF₂, OCF=CF₂, OCF=CF-CF₃, OCH=CF-CF₃, CH=CF₂, CH₂CF₃ oder OCHFCHF₂, insbesondere F, OCF₃, OCHF₂, OCH₂CF₃, OCH₂CHF₂, CN und OCH=CF₂. Vorzugsweise hat der Rest X nicht mehr als 3 C-Atome.

n ist vorzugsweise 0 oder 1. R ist vorzugsweise H oder Methyl.

Der Alkylrest R kann geradkettig oder verzweigt sein. Vorzugsweise ist er geradkettig, hat 1, 2, 3, 4 oder 5 C-Atome und bedeutet demnach bevorzugt Ethyl, Propyl ferner Butyl, Pentyl oder Hexyl.

Oxaalkyl bedeutet vorzugsweise geradkettiges 2-Oxapropyl (= Methoxymethyl), 2- (=Ethoxymethyl) oder 3-Oxabutyl (= 2-Methoxyethyl), 2-, 3- oder 4-Oxapentyl, 2-, 3-, 4- oder 5-Oxahexyl, 2-, 3-, 4-, 5- oder 6-Oxaheptyl. Falls R einen Alkylrest bedeutet, in dem eine CH2-Gruppe durch -CH=CH-ersetzt ist, so kann dieser geradkettig oder verzweigt sein. Vorzugsweise ist er geradkettig und hat 2 bis 7 C-Atome. Er bedeutet demnach besonders Vinyl, Prop-1-, oder Prop-2-enyl, But-1-, 2- oder But-3-enyl, Pent-1-, 2-, 3- oder Pent-4-enyl, Hex-1-, 2-, 3-, 4- oder Hex-5-enyl, Hept-1, 2-, 3-, 4-, 5- oder Hept-6-enyl.

Unter diesen Verbindungen der Formel I sowie den Unterformeln sind diejenigen bevorzugt, in denen mindestens einer der darin enthaltenden Reste eine der angegebenen bevorzugten Bedeutungen hat.

In den Verbindungen der Formel I sind diejenigen Stereoisomeren bevorzugt, in denen der Cyclohexylenring trans-1,4-disubstituiert ist.

Bevorzugte kleinere Gruppen von Verbindungen sind diejenigen der Teilformeln 11 bis 16:

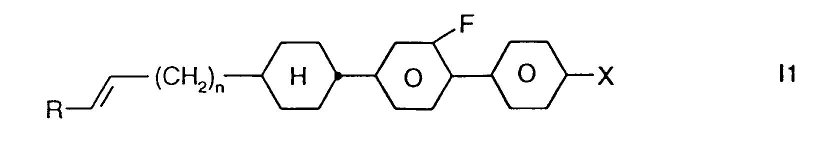

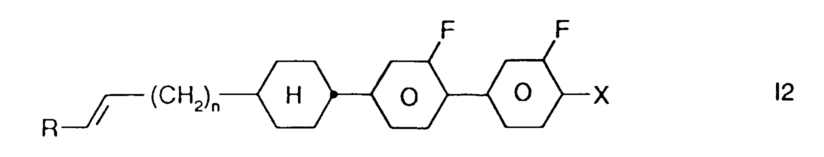

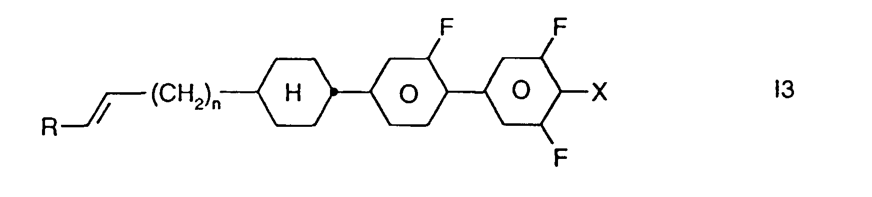

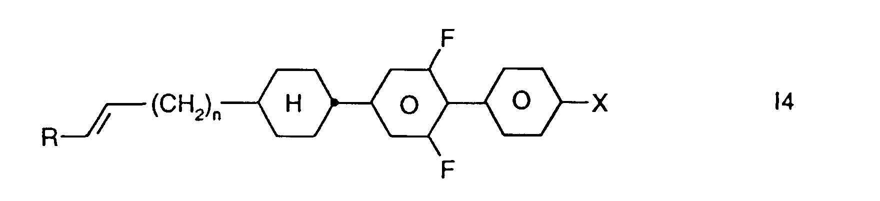

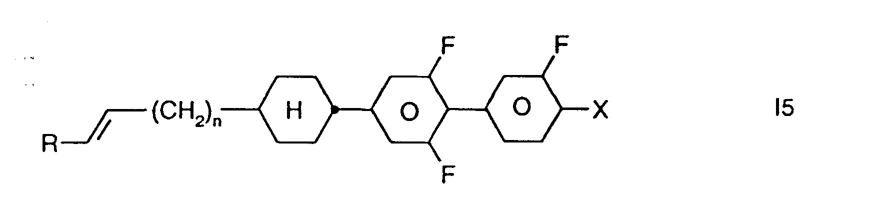

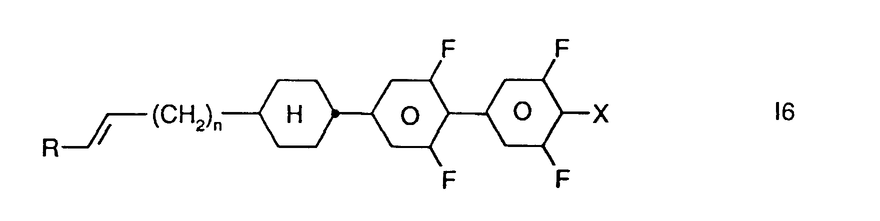

Die Verbindungen der Formel I werden nach an sich bekannten Methoden dargestellt, wie sie in der Literatur (z.B. in den Standardwerken wie Houben-Weyl, Methoden der Organischen Chemie, Georg-Thieme-Verlag, Stuttgart) beschrieben sind, und zwar unter Reaktionsbedingungen, die für die genannten Umsetzungen bekannt und geeignet sind.

Dabei kann man auch von an sich bekannten, hier nicht näher erwähnten Varianten Gebrauch machen.

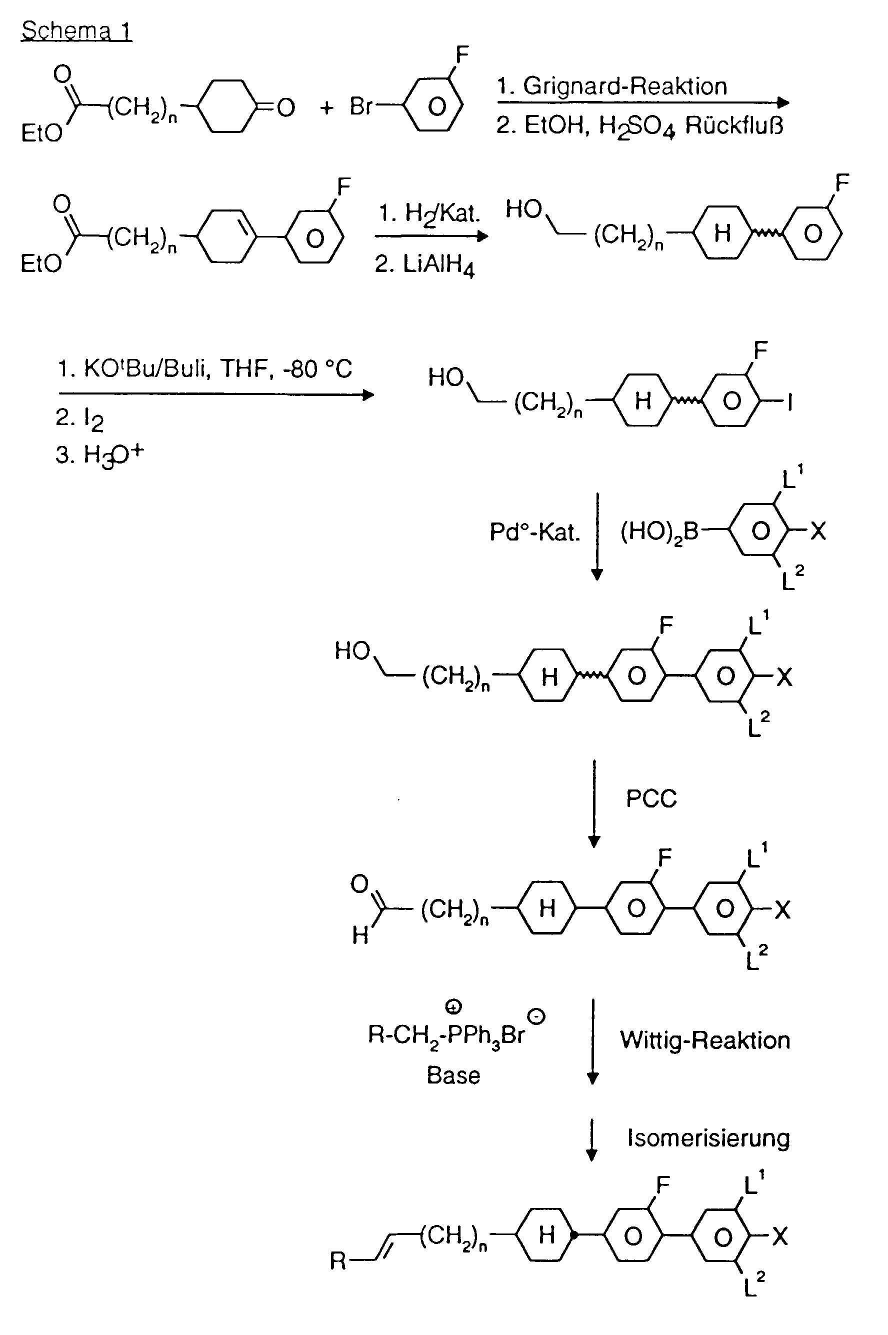

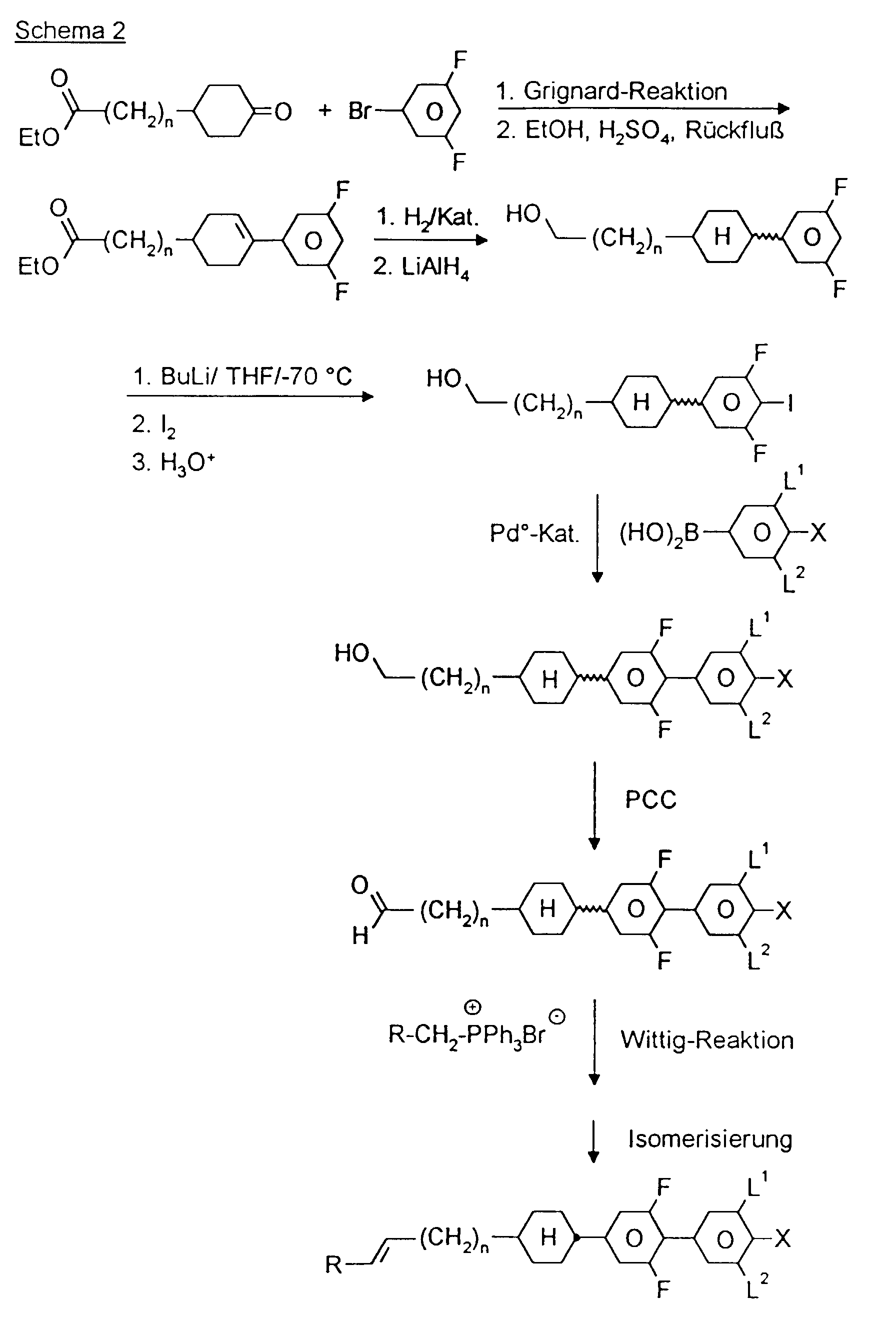

Die erfindungsgemäßen flüssigkristallinen Medien enthalten vorzugsweise neben einer oder mehreren Verbindungen der Formel I als weitere Bestandteile 2 bis 40, insbesondere 4 bis 30 Komponenten. Ganz besonders bevorzugt enthalten diese Medien neben einer oder mehreren erfindungsgemäßen Verbindungen 7 bis 25 Komponenten. Diese weiteren Bestandteile werden vorzugsweise ausgewählt aus nematischen oder nematogenen (monotropen oder isotropen) Substanzen, insbesondere Substanzen aus den Klassen der Azoxybenzole, Benzylidenaniline, Biphenyle, Terphenyle, Phenyl- oder Cyclohexylbenzoate, Cyclohexancarbonsäure-phenyl- oder cyclohexyl-ester, Phenyl- oder Cyclohexyl-ester der Cyclohexylbenzoesäure, Phenyl- oder Cyclohexyl-ester der Cyclohexylcyclohexancarbonsäure, Cyclohexyl-phenylester der Benzoesäure, der Cyclohexancarbonsäure, bzw. der Cyclohexylcyclohexancarbonsäure, Phenylcyclohexane, Cyclohexylbiphenyle, Phenylcyclohexylcyclohexane, Cyclohexylcyclohexane, Cyclohexylcyclohexylcyclohexene, 1,4-Biscyclohexylbenzole, 4,4'-Bis-cyclohexylbiphenyle, Phenyl- oder Cyclohexylpyrimidine, Phenyl- oder Cyclohexylpyridine, Phenyl- oder Cyclohexyldioxane, Phenyl- oder Cyclohexyl-1,3-dithiane, 1,2-Diphenylethane, 1,2-Dicyclohexylethane, 1-Phenyl-2-cyclohexylethane, 1-Cyclohexyl-2-(4-phenylcyclohexyl)-ethane, 1-Cyclohexyl-2-biphenylylethane, 1-Phenyl-2-cyclohexyl-phenylethane, gegebenenfalls halogenierten Stilbene, Benzylphenylether, Tolane und substituierten Zimtsäuren. Die 1,4-Phenylengruppen in diesen Verbindungen können auch fluoriert sein.

Die wichtigsten als weitere Bestandteile erfindungsgemäßer Medien in Frage kommenden Verbindungen lassen sich durch die Formeln 1, 2, 3, 4 und 5 charakterisieren:

R'-L-E-R" 1

R'-L-COO-E-R'' 2

R'-L-OOC-E-R'' 3

R'-L-CH₂CH₂-E-R'' 4

R'-L-C≡C-E-R'' 5

In den Formeln 1, 2, 3, 4 und 5 bedeuten L und E, die gleich oder verschieden sein können, jeweils unabhängig voneinander einen bivalenten Rest aus der aus -Phe-, -Cyc-, -Phe-Phe-, -Phe-Cyc-, -Cyc-Cyc-, -Pyr-, -Dio-, -G-Phe- und -G-Cyc- sowie deren Spiegelbilder gebildeten Gruppe, wobei Phe unsubstituiertes oder durch Fluor substituiertes 1,4-Phenylen, Cyc trans-1,4-Cyclohexylen oder 1,4-Cyclohexylen, Pyr Pyrimidin-2,5-diyl oder Pyridin-2,5-diyl, Dio 1,3-Dioxan-2,5-diyl und G 2-(trans-1,4-Cyclohexyl)-ethyl, Pyrimidin-2,5-diyl, Pyridin-2,5-diyl oder 1,3-Dioxan-2,5-diyl bedeuten.

Vorzugsweise ist einer der Reste L und E Cyc, Phe oder Pyr. E ist vorzugsweise Cyc, Phe oder Phe-Cyc. Vorzugsweise enthalten die erfindungsgemäßen Medien eine oder mehrere Komponenten ausgewählt aus den Verbindungen der Formeln 1, 2, 3, 4 und 5, worin L und E ausgewählt sind aus der Gruppe Cyc, Phe und Pyr und gleichzeitig eine oder mehrere Komponenten ausgewählt aus den Verbindungen der Formeln 1, 2, 3, 4 und 5, worin einer der Reste L und E ausgewählt ist aus der Gruppe Cyc, Phe und Pyr und der andere Rest ausgewählt ist aus der Gruppe -Phe-Phe-, -Phe-Cyc-, -Cyc-Cyc-, -G-Phe- und -G-Cyc-, und gegebenenfalls eine oder mehrere Komponenten ausgewählt aus den Verbindungen der Formeln 1, 2, 3, 4 und 5, worin die Reste L und E ausgewählt sind aus der Gruppe -Phe-Cyc-, -Cyc-Cyc-, -G-Phe- und -G-Cyc-.

R' und R" bedeuten in einer kleineren Untergruppe der Verbindungen der Formeln 1, 2, 3, 4 und 5 jeweils unabhängig voneinander Alkyl, Alkenyl, Alkoxy, Alkoxyalkyl, Alkenyloxy oder Alkanoyloxy mit bis zu 8 Kohlenstoffatomen. Im folgenden wird diese kleinere Untergruppe Gruppe A genannt und die Verbindungen werden mit den Teilformeln 1a, 2a, 3a, 4a und 5a bezeichnet. Bei den meisten dieser Verbindungen sind R' und R" voneinander verschieden, wobei einer dieser Reste meist Alkyl, Alkenyl, Alkoxy oder Alkoxyalkyl ist.

In einer anderen als Gruppe B bezeichneten kleineren Untergruppe der Verbindungen der Formeln 1, 2, 3, 4 und 5 bedeutet R''-F, -Cl, -NCS oder -(O)ᵢCH₃₋₍ₖ₊ₗ₎FₖClₗ, wobei i 0 oder 1 und k+l 1, 2 oder 3 sind; die Verbindungen, in denen R'' diese Bedeutung hat, werden mit den Teilformeln 1b, 2b, 3b, 4b und 5b bezeichnet. Besonders bevorzugt sind solche Verbindungen der Teilformeln 1b, 2b, 3b, 4b und 5b, in denen R'' die Bedeutung -F, -Cl, -NCS, -CF₃, -OCHF₂ oder -OCF₃ hat.

In den Verbindungen der Teilformeln 1b, 2b, 3b, 4b und 5b hat R' die bei den Verbindungen der Teilformeln 1a-5a angegebene Bedeutung und ist vorzugsweise Alkyl, Alkenyl, Alkoxy oder Alkoxyalkyl.

In einer weiteren kleineren Untergruppe der Verbindungen der Formeln 1, 2, 3, 4 und 5 bedeutet R''-CN; diese Untergruppe wird im folgenden als Gruppe C bezeichnet und die Verbindungen dieser Untergruppe werden entsprechend mit Teilformeln 1c, 2c, 3c, 4c und 5 c beschrieben. In den Verbindungen der Teilformeln 1c, 2c, 3c, 4c und 5c hat R' die bei den Verbindungen der Teilformeln 1a-5a angegebene Bedeutung und ist vorzugsweise Alkyl, Alkoxy oder Alkenyl.

Neben den bevorzugten Verbindungen der Gruppen A, B und C sind auch andere Verbindungen der Formeln 1, 2, 3, 4 und 5 mit anderen Varianten der vorgesehenen Substituenten gebräuchlich. Alle diese Substanzen sind nach literaturbekannten Methoden oder in Analogie dazu erhältlich.

Die erfindungsgemäßen Medien enthalten neben erfindungsgemäßen Verbindungen der Formel I vorzugsweise eine oder mehrere Verbindungen, welche ausgewählt werden aus der Gruppe A und/oder Gruppe B und/oder Gruppe C. Die Massenanteile der Verbindungen aus diesen Gruppen an den erfindungsgemäßen Medien sind vorzugsweise
- Gruppe A:: 0 bis 90 %, vorzugsweise 20 bis 90 %, insbesondere 30 bis 90 %
- Gruppe B:: 0 bis 80 %, vorzugsweise 10 bis 80 %, insbesondere 10 bis 65 %
- Gruppe C:: 0 bis 80 %, vorzugsweise 5 bis 80 %, insbesondere 5 bis 50 %

wobei die Summe der Massenanteile der in den jeweiligen erfindungsgemäßen Medien enthaltenen Verbindungen aus den Gruppen A und/oder B und/oder C vorzugsweise 5 bis 90 % und insbesondere 10 bis 90 % beträgt.

Die erfindungsgemäßen Medien enthalten vorzugsweise 1 bis 40 %, insbesondere vorzugsweise 5 bis 30 % an erfindungsgemäßen Verbindungen. Weiterhin bevorzugt sind Medien, enthaltend mehr als 40 %, insbesondere 45 bis 90 % an erfindungsgemäßen Verbindungen. Die Medien enthalten vorzugsweise drei, vier oder fünf erfindungsgemäße Verbindungen.

Vorzugsweise basieren die erfindungsgemäßen Medien auf mehreren (vorzugsweise zwei oder mehr) Verbindungen der Formel I, d.h. der Anteil dieser Verbindungen ist ≥ 25 %, vorzugsweise ≥ 40 %.

Die einzelnen Verbindungen der Formeln I bis XII und deren Unterformeln, die in den erfindungsgemäßen Medien verwendet werden können, sind entweder bekannt, oder sie können analog zu den bekannten Verbindungen hergestellt werden.

Bevorzugte Ausführungsformen sind im folgenden angegeben:
- Medium enthält zusätzlich eine oder mehrere Verbindungen ausgewählt aus der Gruppe bestehend aus den allgemeinen Formeln V bis VIII: 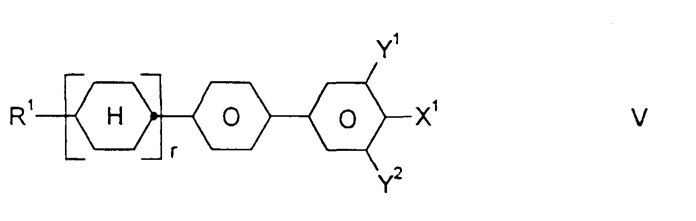 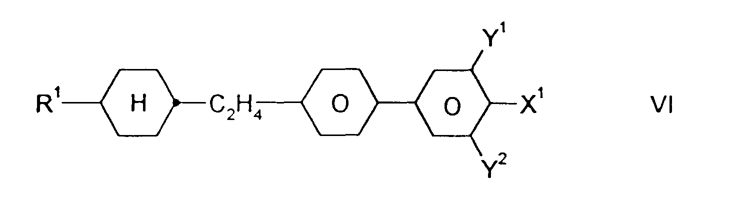 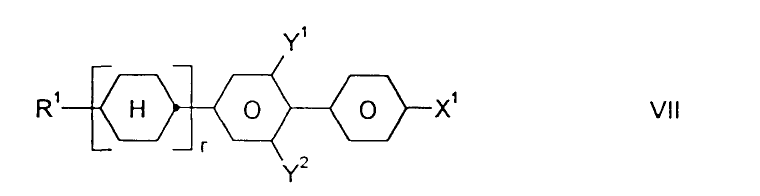 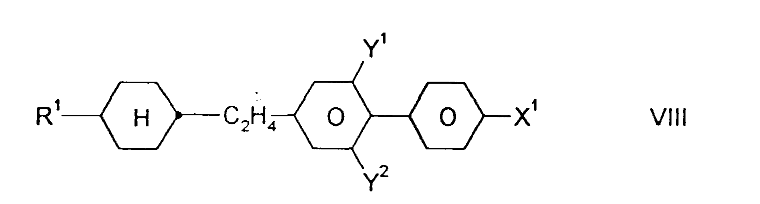 worin R¹, X¹, Y¹ und Y² jeweils unabhängig voneinander die oben angegebene Bedeutung haben.
- Medium enthält zusätzlich eine oder mehrere Verbindungen ausgewählt aus der Gruppe bestehend aus den allgemeinen Formeln IX bis XII: 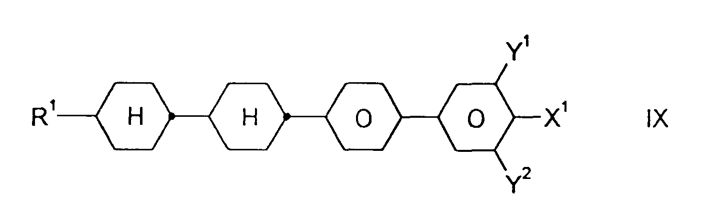 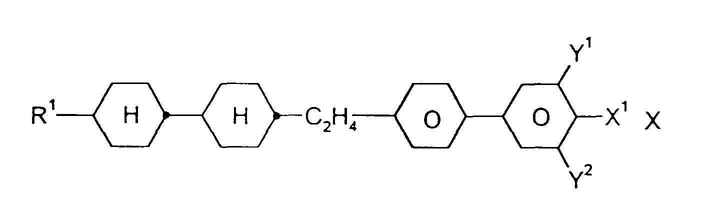 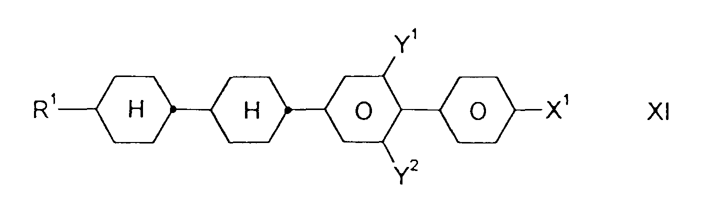 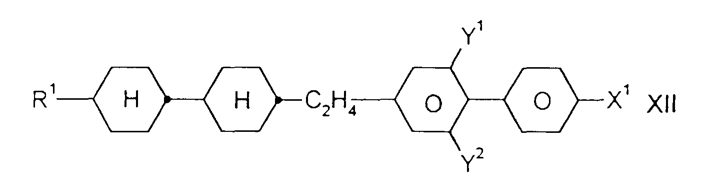 worin R¹, X¹, Y¹ und Y² jeweils unabhängig voneinander die oben angegebene Bedeutung haben.
- Der Anteil an Verbindungen der Formeln I bis IV zusammen beträgt im Gesamtgemisch mindestens 50 Gew.%
- Der Anteil an Verbindungen der Formel I beträgt im Gesamtgemisch 10 bis 50 Gew.%
- Der Anteil an Verbindungen der Formeln II bis IV im Gesamtgemisch beträgt 30 bis 70 Gew.% 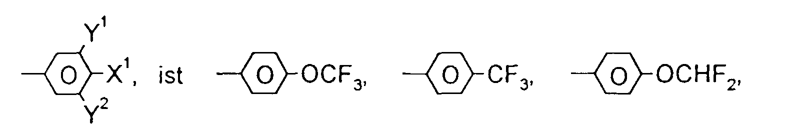 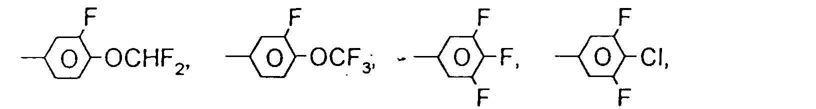 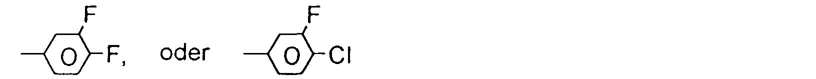
- Das Medium enthält Verbindungen der Formeln II und III oder IV
- R¹ ist geradkettiges Alkyl oder Alkenyl mit 2 bis 7 C-Atomen
- Das Medium besteht im wesentlichen aus Verbindungen der Formeln I bis IV
- Das Medium enthält weitere Verbindungen, vorzugsweise ausgewählt aus der folgenden Gruppe (Y¹, Y², Y³: H oder F) 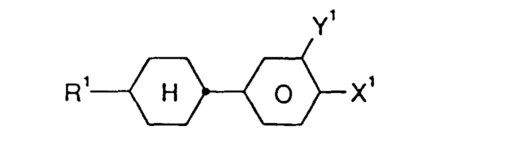 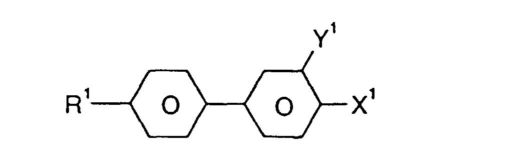 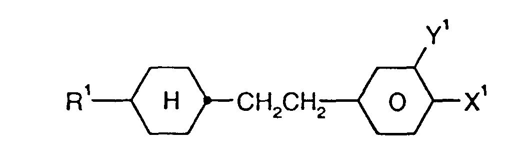 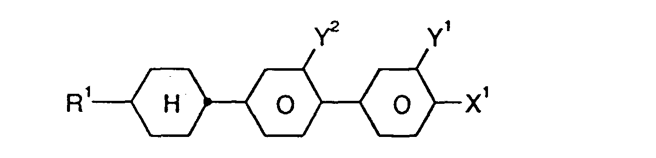 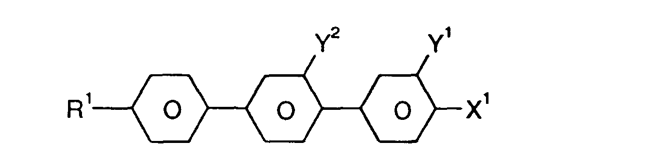 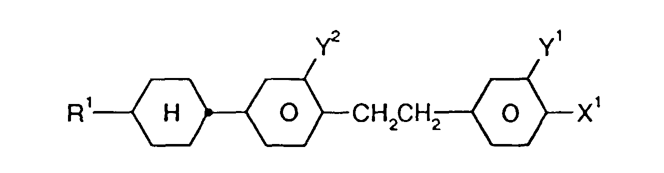 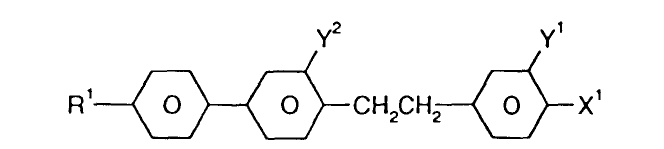 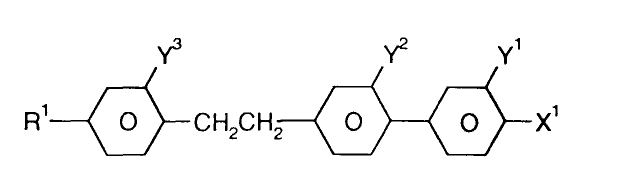 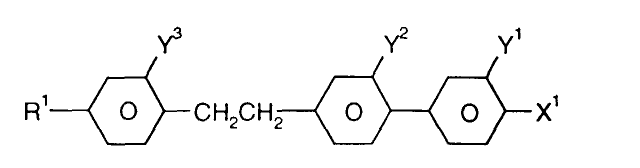 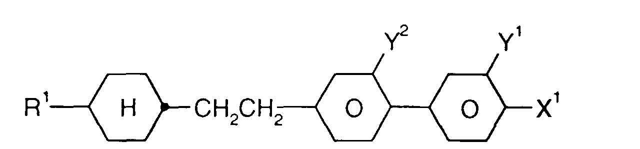
- Das Gewichtsverhältnis I: (II + III + IV) ist vorzugsweise 1 : 4 bis 1 : 1.
- Medium besteht im wesentlichen aus Verbindungen ausgewählt aus der Gruppe bestehend aus den allgemeinen Formeln I bis XII.

Es wurde gefunden, daß bereits ein relativ geringer Anteil an Verbindungen der Formel I im Gemisch mit üblichen Flüssigkristallmaterialien, insbesondere jedoch mit einer oder mehreren Verbindungen der Formel II, III und/oder IV zu einer beträchtlichen Verbesserung der Ansprechzeiten und zu niedrigen Schwellenspannungen führt, wobei gleichzeitig breite nematische Phasen mit tiefen Übergangstemperaturen smektisch-nematisch beobachtet werden. Die Verbindungen der Formeln I bis IV sind farblos, stabil und untereinander und mit anderen Flüssigkristallmaterialien gut mischbar.

Der Ausdruck "Alkyl" umfaßt geradkettige und verzweigte Alkylgruppen mit 1-7 Kohlenstoffatomen, insbesondere die geradkettigen Gruppen Methyl, Ethyl, Propyl, Pentyl, Hexyl und Heptyl. Gruppen mit 2-5 Kohlenstoffatomen sind im allgemeinen bevorzugt.

Der Ausdruck "Alkenyl" umfaßt geradkettige und verzweigte Alkenylgruppen mit 2-7 Kohlenstoffatomen, insbesondere die geradkettigen Gruppen. Besonders Alkenylgruppen sind C₂-C₇-1E-Alkenyl, C₂-C₇-3E-Alkenyl, C₅-C₇-4-Alkenyl, C₆-C₇-5-Alkenyl und C₇-6-Alkenyl, insbesondere C₂-C₇-1E-Alkenyl, C₄-C₇-3E-Alkenyl und C₅-C₇-4-Alkenyl. Beispiele bevorzugter Alkenylgruppen sind Vinyl, 1E-Propenyl, 1E-Butenyl, 1E-Pentenyl, 1E-Hexenyl, 1 E-Heptenyl, 3-Butenyl, 3E-Pentenyl, 3E-Hexenyl, 3E-Heptenyl, 4-Pentenyl, 4Z-Hexenyl, 4E-Hexenyl, 4Z-Heptenyl, 5-Hexenyl, 6-Heptenyl und dergleichen. Gruppen mit bis zu 5 Kohlenstoffatomen sind im allgemeinen bevorzugt.

Der Ausdruck "Fluoralkyl" umfaßt vorzugsweise geradkettige Gruppen mit endständigen Fluor, d.h. Fluormethyl, 2-Fluorethyl, 3-Fluorpropyl, 4-Fluorbutyl, 5-Fluorpentyl, 6-Fluorhexyl und 7-Fluorheptyl. Andere Positionen des Fluor sind jedoch nicht ausgeschlossen.

Der Ausdruck "Oxaalkyl" umfaßt vorzugsweise geradkettige Reste der Formel CₙH₂ₙ₊₁-O-(CH₂)ₘ, worin n und m jeweils unabhängig voneinander 1 bis 6 bedeuten. Vorzugsweise ist n = 1 und m 1 bis 6.

Durch geeignete Wahl der Bedeutungen von R, X und Y können die Ansprechzeiten, die Schwellenspannung, die Steilheit der Transmissionskennlinien etc. in gewünschter Weise modifiziert werden. Beispielsweise führen 1 E-Alkenylreste, 3E-Alkenylreste, 2E-Alkenyloxyreste und dergleichen in der Regel zu kürzeren Ansprechzeiten, verbesserten nematischen Tendenzen und einem höheren Verhältnis der elastischen Konstanten k₃₃ (bend) und k₁₁ (splay) im Vergleich zu Alkyl- bzw. Alkoxyresten. 4-Alkenylreste, 3-Alkenylreste und dergleichen ergeben im allgemeinen tiefere Schwellenspannungen und kleinere Werte von k₃₃/k₁₁ im Vergleich zu Alkyl- und Alkoxyresten.

Eine Gruppe -CH₂CH₂- in Z¹ bzw. Z² führt im allgemeinen zu höheren Werten von k₃₃/k₁₁ im Vergleich zu einer einfachen Kovalenzbindung. Höhere Werte von k₃₃/k₁₁ ermöglichen z.B. flachere Transmissionskennlinien in TN-Zellen mit 90° Verdrillung (zur Erzielung von Grautönen) und steilere Transmissionskennlinien in STN-, SBE- und OMI-Zellen (höhere Multiplexierbarkeit) und umgekehrt.

Das optimale Mengenverhältnis der Verbindungen der Formeln I und II + III + IV hängt weitgehend von den gewünschten Eigenschaften, von der Wahl der Komponenten der Formeln I, II, III und/oder IV und von der Wahl weiterer gegebenenfalls vorhandener Komponenten ab. Geeignete Mengenverhältnisse innerhalb des oben angegebenen Bereichs können von Fall zu Fall leicht ermittelt werden.

Die Gesamtmenge an Verbindungen der Formeln I bis XII in den erfindungsgemäßen Gemischen ist nicht kritisch. Die Gemische können daher eine oder mehrere weitere Komponenten enthalten zwecks Optimierung verschiedener Eigenschaften. Der beobachtete Effekt auf die Ansprechzeiten und die Schwellenspannung ist jedoch in der Regel umso größer je höher die Gesamtkonzentration an Verbindungen der Formeln I bis XII ist.

In einer besonders bevorzugten Ausführungsform enthalten die erfindungsgemäßen Medien Verbindungen der Formel II, III, V und/oder VII (vorzugsweise II und/oder III), worin Xi CF₃, OCF₃, OCHF₂ oder F bedeutet.

Die Herstellung der erfindungsgemäßen Medien erfolgt in an sich üblicher Weise. In der Regel werden die Komponenten ineinander gelöst, zweckmäßig bei erhöhter Temperatur. Durch geeignete Zusätze können die flüssigkristallinen Phasen nach der Erfindung so modifiziert werden, daß sie in allen bisher bekannt gewordenen Arten von Flüssigkristallanzeigeelementen verwendet werden können. Derartige Zusätze sind dem Fachmann bekannt und in der Literatur ausführlich beschrieben (H. Kelker/R. Hatz, Handbook of Liquid Crystals, Verlag Chemie, Weinheim, 1980). Beispielsweise können pleochroitische Farbstoffe zur Herstellung farbiger Guest-Host-Systeme oder Substanzen zur Veränderung der dielektrischen Anisotropie, der Viskosität und/oder der Orientierung der nematischen Phasen zugesetzt werden.

In der vorliegenden Anmeldung und in den folgenden Beispielen sind die Strukturen der Flüssigkristallverbindungen durch Acronyme angegeben, wobei die Transformation in chemische Formeln gemäß folgender Tabellen A und B erfolgt. Alle Reste CₙH₂ₙ₊₁ und CₘH₂ₘ₊₁ sind geradkettige Alkylreste mit n bzw. m C-Atomen. Die Codierung gemäß Tabelle B versteht sich von selbst. In Tabelle A ist nur das Acronym für den Grundkörper angegeben. Im Einzelfall folgt getrennt vom Acronym für den Grundkörper mit einem Strich ein Code für die Substituenten R¹, R², L¹ und L²:

| Code für R¹, R², L¹, L² | R¹ | R² | L¹ | L² |
|---|---|---|---|---|
| nm | CₙH₂ₙ₊₁ | CₘH₂ₘ₊₁ | H | H |
| nOm | CₙH₂ₙ₊₁ | OCₘH₂ₘ₊₁ | H | H |
| nO.m | OCₙH₂ₙ₊₁ | CₘH₂ₘ₊₁ | H | H |
| n | CₙH₂ₙ₊₁ | CN | H | H |
| nN.F | CₙH₂ₙ₊₁ | CN | H | F |
| nF | CₙH₂ₙ₊₁ | F | H | H |
| nOF | OCₙH₂ₙ₊₁ | F | H | H |
| nCl | CₙH₂ₙ₊₁ | Cl | H | H |
| nF.F | CₙH₂ₙ₊₁ | F | H | F |
| nF.F.F | CₙH₂ₙ₊₁ | F | F | F |
| nCF₃ | CₙH₂ₙ₊₁ | CF₃ | H | H |
| nOCF₃ | CₙH₂ₙ₊₁ | OCF₃ | H | H |
| nOCF₂ | CₙH₂ₙ₊₁ | OCHF₂ | H | H |
| nS | CₙH₂ₙ₊₁ | NCS | H | H |
| rVsN | CᵣH₂ᵣ₊₁-CH=CH-CₛH₂ₛ- | CN | H | H |
| rEsN | CᵣH₂ᵣ₊₁-O-CₛH₂ₛ- | CN | H | H |
| nAm | CₙH₂ₙ₊₁ | COOCₘH₂ₘ₊₁ | H | H |

Bevorzugte Mischungen enthalten neben ein oder mehreren Verbindungen der Formel I Verbindungen aus den Tabellen A und B.

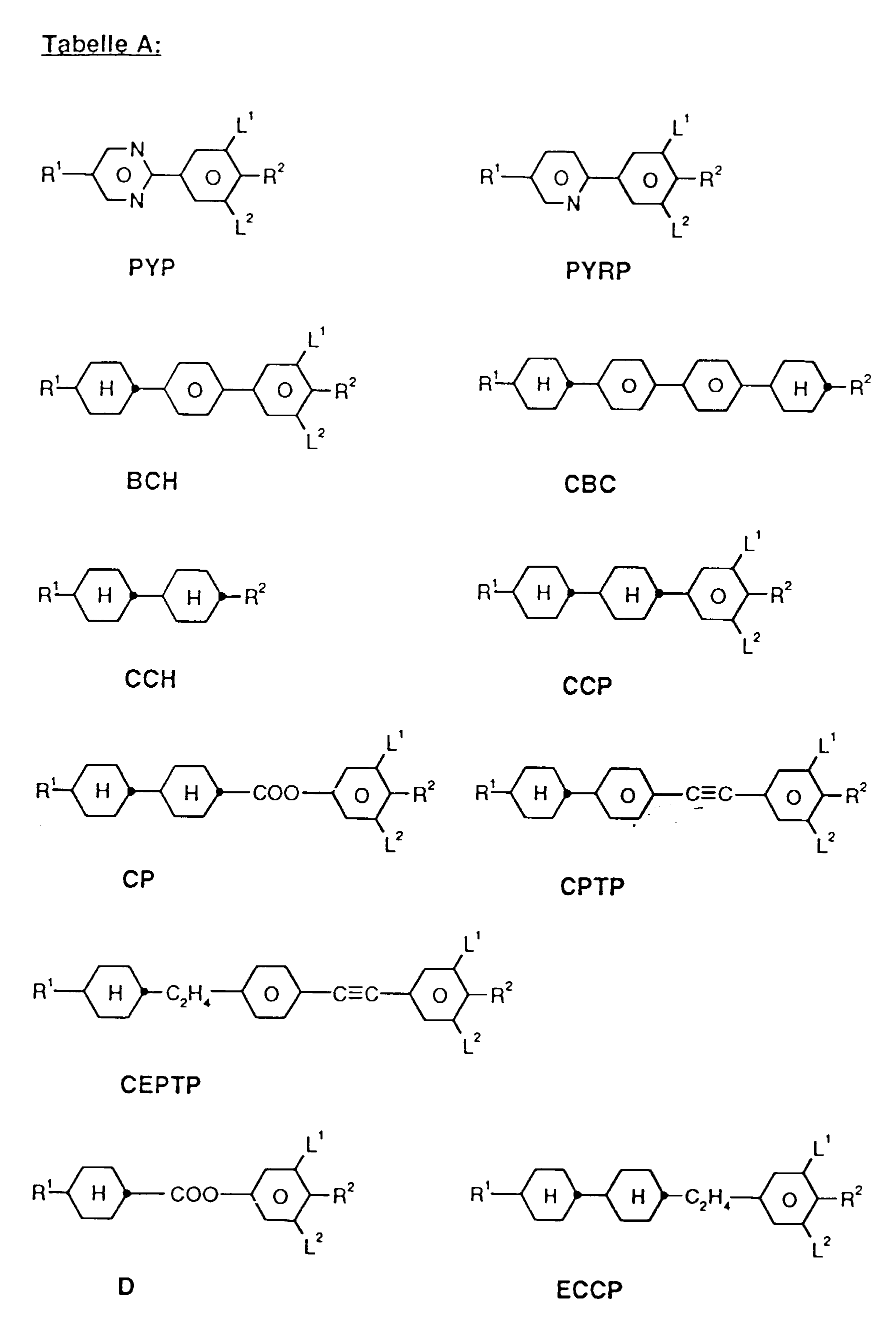

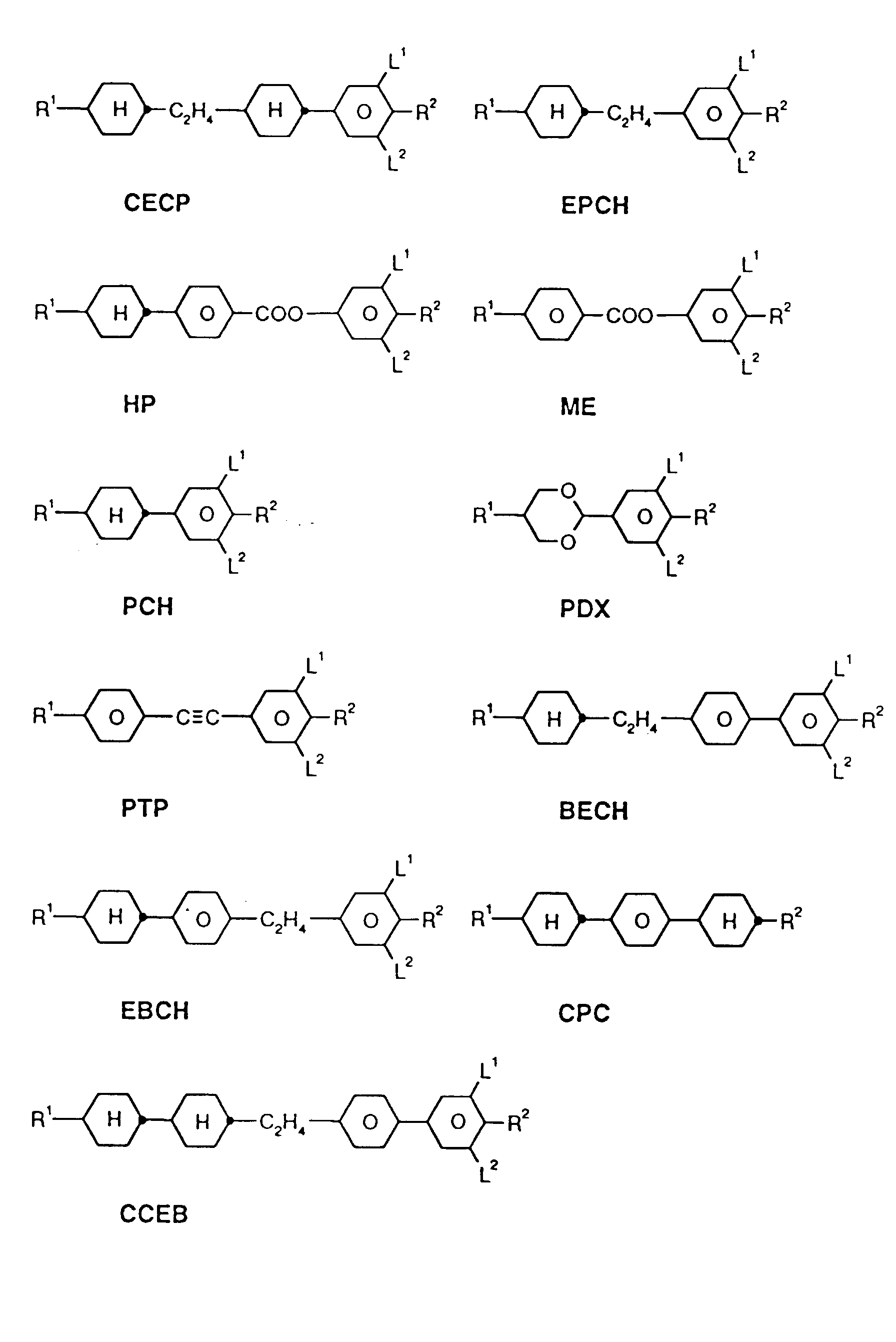

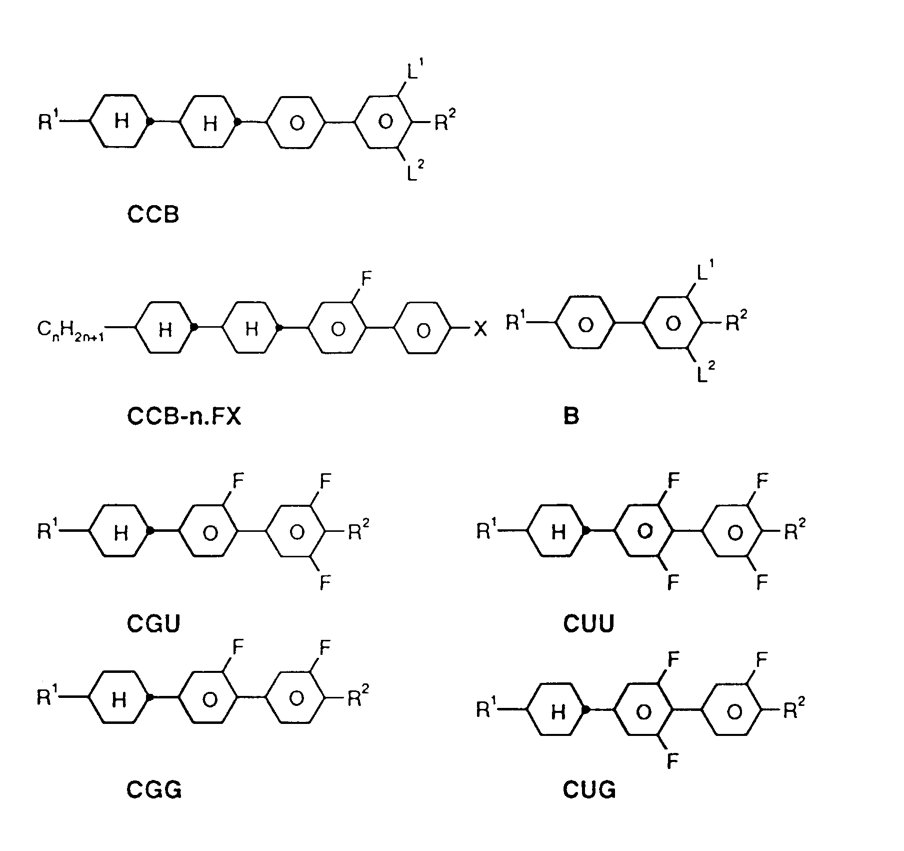

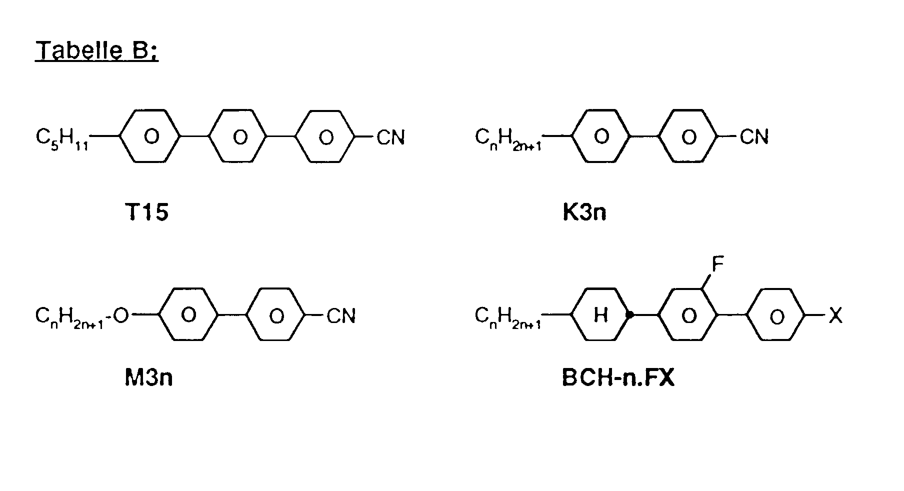

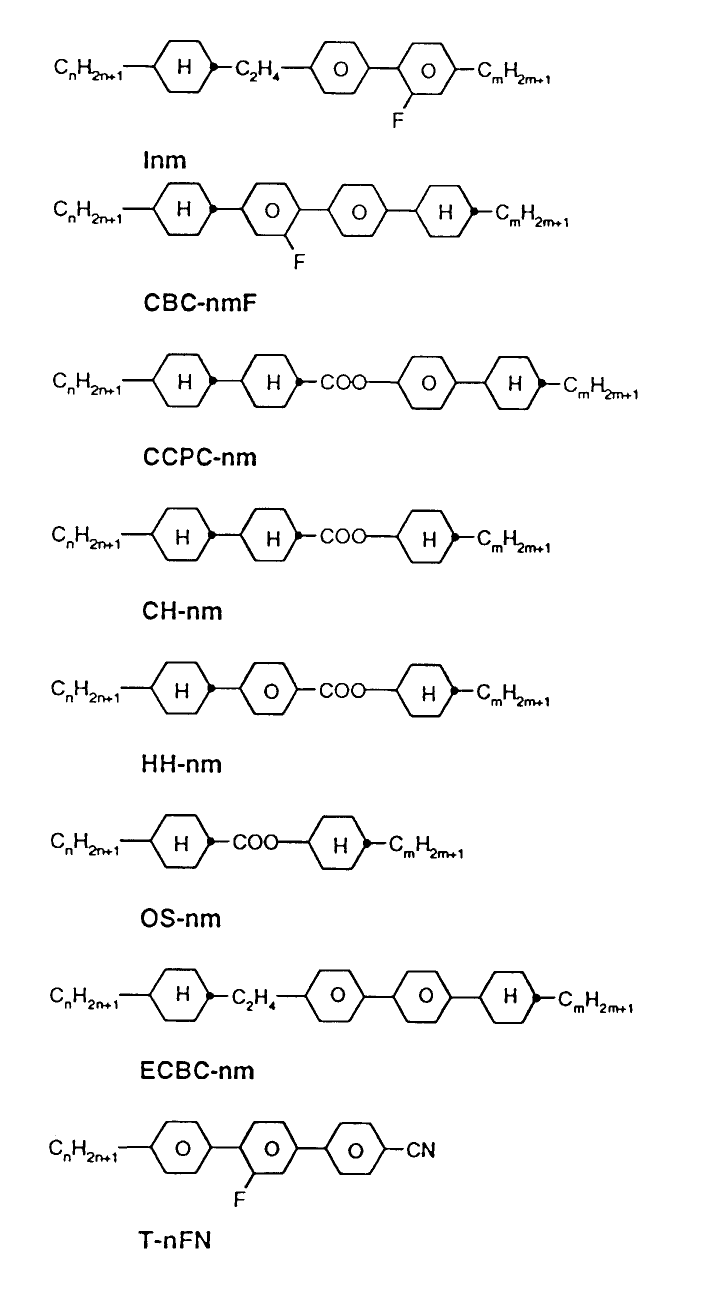

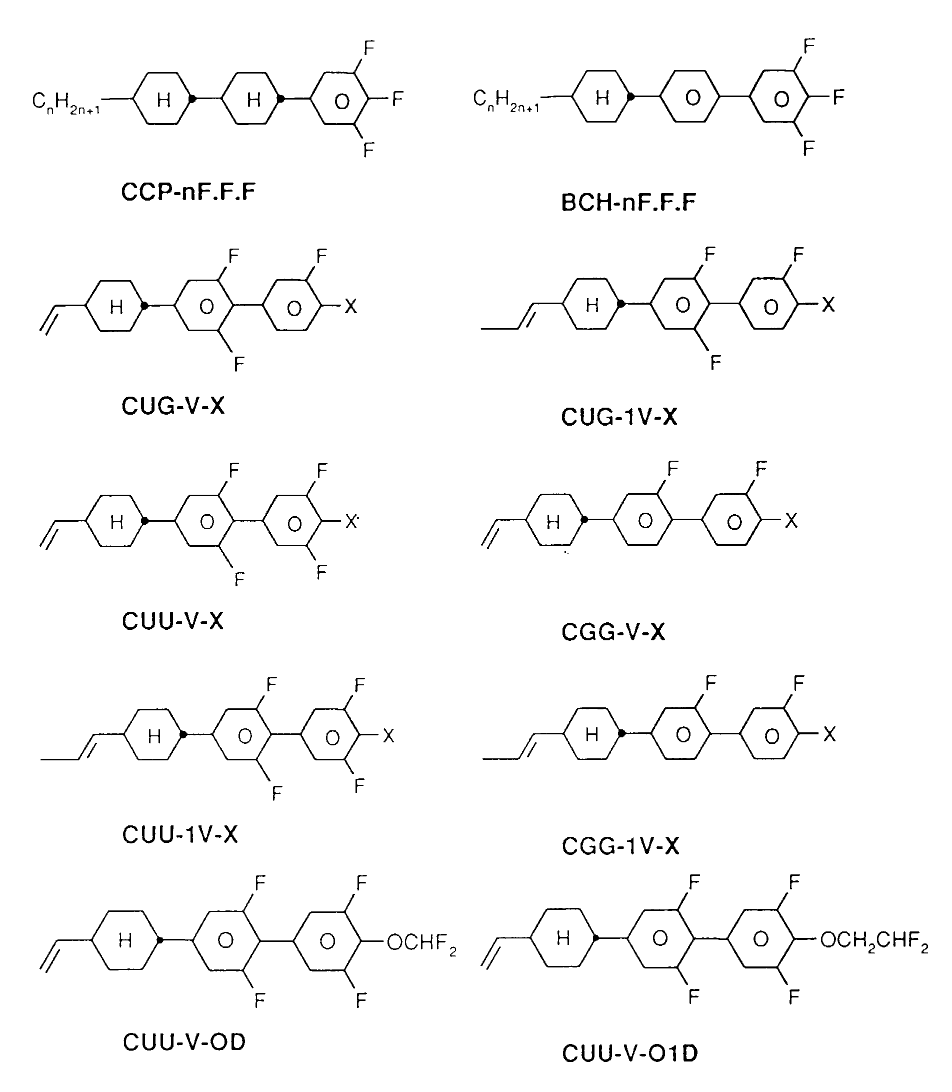

Die folgenden Beispiele sollen die Erfindung erläutern, ohne sie zu begrenzen. Vor- und nachstehend bedeuten Prozentangaben Gewichtsprozent. Alle Temperaturen sind in Grad Celsius angegeben. Fp. bedeutet Schmelzpunkt, Kp. = Klärpunkt. Ferner bedeuten K = kristalliner Zustand, N = nematische Phase, S = smektische Phase und I = isotrope Phase. Die Angaben zwischen diesen Symbolen stellen die Übergangstemperaturen dar. Δn bedeutet optische Anisotropie (589 nm, 20 °C) und die Viskosität (mm²/sec) wurde bei 20 °C bestimmt.

"Übliche Aufarbeitung" bedeutet: man gibt gegebenenfalls Wasser hinzu, extrahiert mit Dichlormethan, Diethylether, Methyl-tert.Butylether oder Toluol, trennt ab, trocknet die organische Phase, dampft ein und reinigt das Produkt durch Destillation unter reduziertem Druck oder Kristallisation und/oder Chromatographie. Folgende Abkürzungen werden verwendet:

| | |
|---|---|
| DAST | Diethylaminoschwefeltrifluorid |
| DMEU | 1,3-Dimethyl-2-imidazolidinon |
| KOT | Kalium-tertiär-butanolat |
| THF | Tetrahydrofuran |
| pTsOH | p-Toluolsulfonsäure |

### Beispiel 1

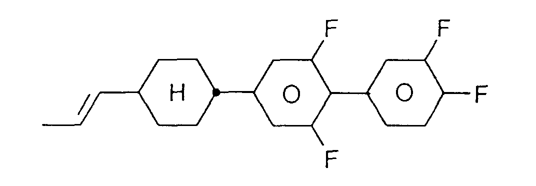

### Schritt 1.1

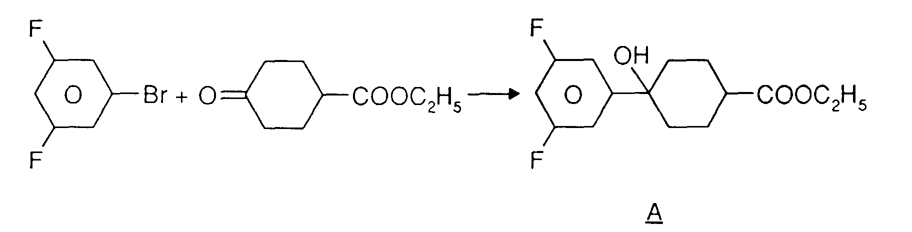

Zu 0,075 mol Magnesiumspäne werden in einer Stickstoffatmosphäre 0,05 mol 3,5-Difluorbenzol in 50 ml THF zugetropft. Anschließend wird 1 h am Rückfluß gekocht. Man läßt auf Raumtemperatur abkühlen und tropft 0,05 mol 4-Cyclohexanoncarbonsäuremethylester gelöst in 50 ml THF zu dem Reaktionsgemisch, so daß die Innentemperatur 30 °C nicht übersteigt. Nach 1 h Rühren wird hydrolysiert und zuletzt wie üblich aufgearbeitet.

### Schritt 1.2

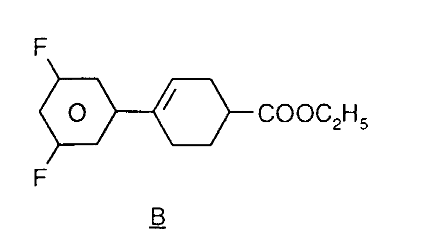

1,4 mol A werden in 2 l Ethanol und 100 ml konz. Schwefelsäure gelöst und 4 h am Rückfluß gekocht. Das Ethanol wird weitgehenst abdestilliert und den Rückstand läßt man auf Raumtemperatur abkühlen. Nach Zugabe von Wasser und Methyl-tert. Butylether wird wie üblich aufgearbeitet.

### Schritt 1.3

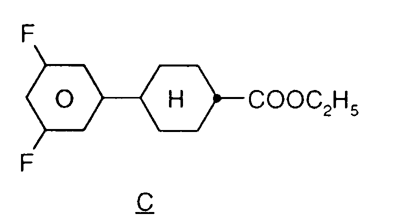

1,13 mol B werden in 2,5 l THF gelöst und mit 35 g Pd/C-Katalysator bei Raumtemperatur hydriert. Nach beendeter Hydrierung wird der Katalysator abfiltriert und das Filtrat fraktioniert destilliert.
Kp.: 90-120 °C (2-10⁻⁵ mbar)

### Schritt 1.4

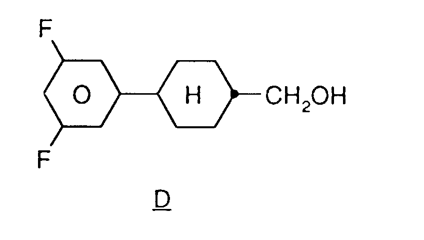

0,12 mol C werden in 100 ml THF in einer Stickstoffatmosphäre gelöst und bei 0 °C mit 0,12 mol Boran-THF-Komplex (1 molare Lösung) versetzt. Nach 1 h Rühren bei 0 °C läßt man auf Raumtemperatur erwärmen und rührt über Nacht. Das Reaktionsgemisch wird mit H₂O/THF (1:1) hydrolysiert und wie üblich aufgearbeitet.

### Schritt 1.5

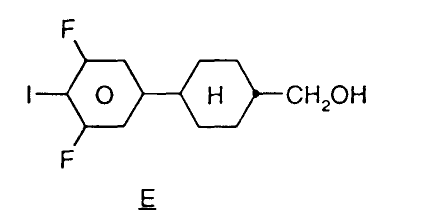

88 mmol D werden in einer Stickstoffatmosphäre in 100 ml THF gelöst und bei -70 °C mit 0,176 mol BuLi (1,6 molare Lösung in n-Hexan) versetzt. Man rührt 1 h bei -70 °C nach und tropft bei -30 °C eine Lösung von 88 mmol lod in 100 ml THF zu dem Reaktionsgemisch zu. Nach beendeter Zugabe wird eine weitere Stunde bei -25 bis -30 °C nachgerührt. Anschließend läßt man auf Raumtemperatur erwärmen, versetzt mit Wasser, verd. HCI und einer Natriumhydrogensulfitlösung. Das Reaktionsgemisch wird zuletzt wie üblich aufgearbeitet.

### Schritt 1.6

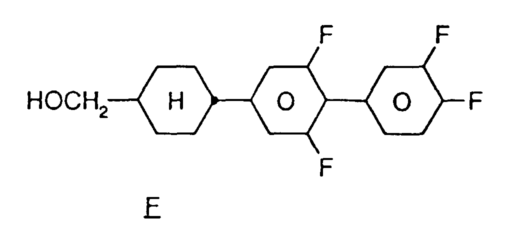

0,086 mol E, 0,086 mol 3,4-Difluorbenzolboronsäure, 0,170 mol Natriumcarbonat in 120 ml Wasser, 200 ml Ethanol, 120 ml Toluol und 0,5 g Tetrakis(triphenylphosphin)palladium(0) werden 4 h am Rückfluß gekocht. Man läßt das Reaktionsgemisch auf Raumtemperatur abkühlen und arbeitet wie üblich auf.

### Schritt 1.7

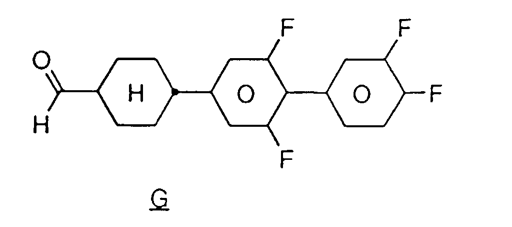

78 mmol F werden in 200 ml Dichlormethan gelöst und bei Raumtemperatur mit 99 mmol Pyridiniumchlorochromat und 13,7 g Celite versetzt und über Nacht gerührt. Nach Zugabe von 10 ml Isopropanol wird 0,5 h gerührt und das Celite abgenutscht. Das Filtrat wird mit Wasser verdünnt und wie üblich aufgearbeitet.

### Schritt 1.8

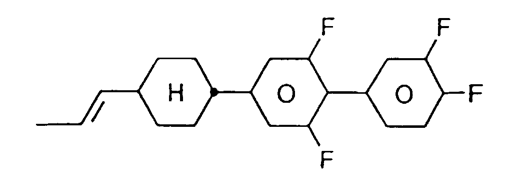

38,7 mmol G, 38,7 mmol Ethyltriphenylphosphoniumbromid in 100 ml THF werden vorgelegt und portionsweise mit 4,3 g Kalium-tert.butylat bei 15-20 °C versetzt. Man rührt 2 h bei Raumtemperatur, versetzt mit Wasser und verd. HCl und arbeitet wie üblich auf. Das Produkt wird aus Essigsäureethylester/Hexan (1:1) umkristallisiert.

18,1 mmol des Produkts, 4,7 mmol Benzolsulfinsäure-Natriumsalz, 7,2 mmol 1 N HCI, 30 ml Toluol und 15 ml THF werden für die E/Z-lsomerisierung 48 h am Rückfluß gekocht. Nach dem Abkühlen des Reaktionsgemisches wird mit 2 molarer Natriumcarbonatlösung alkalisch eingestellt, die wäßrige Phase abgetrennt und wie üblich aufgearbeitet. Das Produkt wird aus Ethanol umkristallisiert.
K 113 I; Δn = +0,114; Δε = 12,73

### Beispiel 2

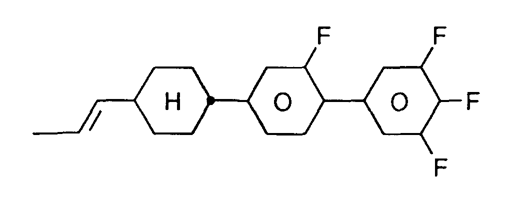

### Schritt 2.1

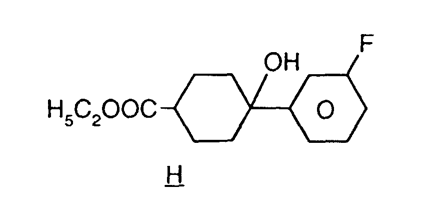

1,1 mol Magnesiumspäne in 100 ml THF werden vorgelegt und erwärmt. Anschließend wird eine Lösung aus 1,1 mol 1-Brom-3-fluorbenzol und 700 ml THF unter Rückfluß zugetropft. Es wird 1 h unter Rückfluß gekocht. Man kühlt auf 10 °C ab und tropft bei 10-20 °C 1,0 mol 4-Cyclohexanon-carbonsäureethylester in 400 ml THF zu dem Reaktionsgemisch. Es wird 0,5 h bei 20 °C nachgerührt und anschließend mit 50 ml Wasser und 200 ml Methyl-tert. Butylether versetzt. Zuletzt wird wie üblich aufgearbeitet.

### Schritt 2.2

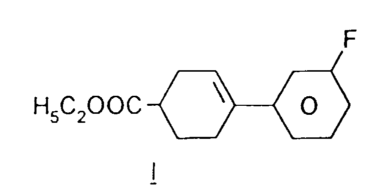

0,75 mol H, 1125 ml Ethanol und 75 ml Schwefelsäure werden 4 h unter Rückfluß gekocht. Das Reaktionsgemisch wird am Rotavapor eingeengt und der Rückstand wird mit Wasser und Methyl-tert. Butylether versetzt. Zuletzt wird wie üblich aufgearbeitet.

### Schritt 2.3

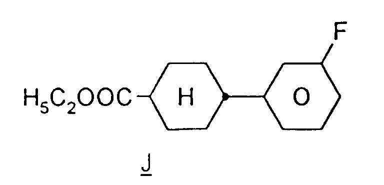

0,74 mol I werden in 1,8 l THF gelöst, mit 35 g Pd/C (5 %) versetzt und hydriert. Nach beendeter Hydrierung wird der Katalysator abfiltriert und, das Filtrat wird am Rotavapor eingeengt. Der Rückstand wird fraktioniert destilliert.
Kp_{0,3}: 103-113 °C

### Schritt 2.4

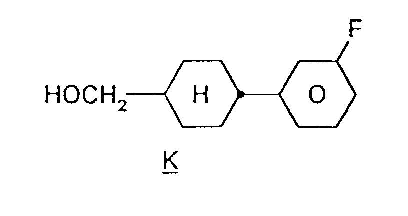

Zu 0,82 mol Lithiumaluminiumhydrid in 1,1 l Diethylether werden 0,82 mol J bei 5-15 °C zugetropft. Nach beendeter Zugabe wird 0,5 h gerührt und anschließend mit Wasser und 20%iger Schwefelsäure hydrolysiert. Man rührt weitere 0,5 h und arbeitet wie üblich auf.

### Schritt 2.5

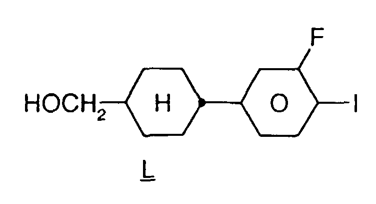

Zu 0,2 mol Kalium-tert.butylat in 100 ml THF werden bei -80 °C 0,1 mol K gelöst in 100 ml THF zugetropft. Anschließend werden bei -80 °C 0,2 mol Butyllithium (15 % in n-Hexan) zu dem Reaktionsgemisch zugetropft. Man rührt 0,5 h nach, verdünnt mit 100 ml THF und tropft 0,1 mol lod in 100 ml THF zu dem Reaktionsgemisch. Anschließend läßt man auf Raumtemperatur erwärmen, hydrolysiert und arbeitet wie üblich auf.

### Schritt 2.6

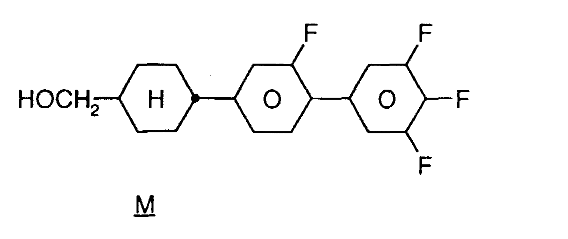

0,19 mol L werden in 200 ml Toluol und nacheinander mit 0,19 mol Boronsäure in 400 ml Ethanol und 0,38 mol Natriumcarbonat in 240 ml Wasser versetzt. Zuletzt wird dem Reaktionsgemisch 5,7 mmol Tetrakis(triphenylphosphin)palladium(0) zugesetzt. Das Reaktionsgemisch wird über Nacht unter Rückfluß gekocht. Man läßt auf Raumtemperatur abkühlen, versetzt mit Toluol und arbeitet wie üblich auf.

### Schritt 2.7

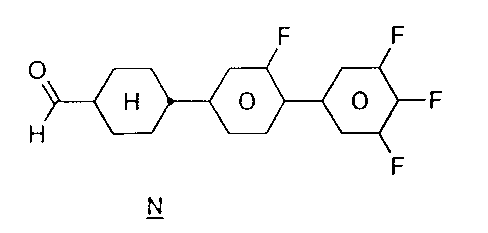

0,19 mol M in 570 ml Dichlormethan werden mit 0,198 mol Pyridiniumchlorochromat und 32 g Celite versetzt und 18 h bei Raumtemperatur gerührt. Nach Zugabe von 30 ml Isopropanol wird weitere 0,5 h gerührt. Celite wird abgesaugt und mit 75 ml Dichlormethan gewaschen. Das Filtrat wird wie üblich aufgearbeitet.

### Schritt 2.8

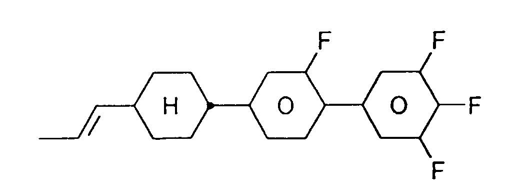

0,1 mol N, 0,1 mol Ethyltriphenylphosphoniumbromid und 240 ml Tetrahydrofuran werden bei 15-20 °C portionsweise mit 0,1 mol Kalium-tert. butylat versetzt. Das Reaktionsgemisch wird 2 h bei Raumtemperatur gerührt. Nach Zugabe von 100 ml Wasser wird mit 2 N HCI auf pH = 7 eingestellt. Anschließend wird wie üblich aufgearbeitet.

Das Produkt (42 mmol) wird in 70 ml Toluol und 35 ml Tetrahydrofuran gelöst und mit 10,9 mmol Benzolsulfinsäure-Natriumsalz und 16,7 ml 1 N HCI versetzt und 4 Tage unter Rückfluß gekocht. Man läßt auf Raumtemperatur abkühlen, gibt 25 ml 2 molare Natriumcarbonatlösung zu dem Reaktionsgemisch und arbeitet wie üblich auf.
K 94 I; Δn = +0,136; Δε = 15,26

Analog werden die folgenden Verbindungen der Formel 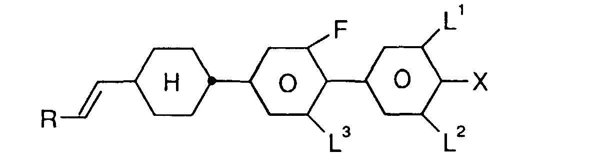 hergestellt:

| R | L¹ | L² | L³ | X | |
|---|---|---|---|---|---|
| H | H | H | H | F | |
| H | F | H | H | F | K66 N (41,2) I; |
| | | | | | Δn = +0,132; Δε = 7,09 |
| H | H | H | F | F | |
| H | F | H | F | F | K 92 I, Δn = +0,111; |
| | | | | | Δε = 10,64 |
| H | F | F | F | F | K139 I |
| H | F | F | H | F | K 82 I, ΔN = +0,115; |
| | | | | | Δε = 12,82 |
| CH₃ | H | H | H | F | |
| CH₃ | H | H | F | F | |
| CH₃ | F | H | H | F | K 67 N 93,4 I; Δn = +0,155; |
| | | | | | Δε = 10,46 |
| CH₃ | F | F | F | F | K 157 I |
| C₂H₅ | H | H | H | F | |
| C₂H₅ | H | H | F | F | |
| C₂H₅ | F | F | F | F | |
| C₂H₅ | F | H | F | F | |
| H | H | H | H | Cl | |
| H | H | H | F | Cl | |
| H | F | F | F | Cl | |
| H | F | H | F | Cl | |
| CH₃ | H | H | H | Cl | |
| CH₃ | H | H | F | Cl | |
| CH₃ | F | F | F | Cl | |
| R | L¹ | L² | L³ | X | |
| C₂H₅ | H | H | H | Cl | |
| C₂H₅ | H | H | F | Cl | |
| C₂H₅ | F | F | F | Cl | |
| C₂H₅ | F | H | F | Cl | |
| H | H | H | H | CN | |
| H | H | H | F | CN | |
| H | F | F | F | CN | |
| H | F | H | F | CN | |
| CH₃ | H | H | H | CN | |
| CH₃ | H | H | F | CN | |
| CH₃ | F | F | F | CN | |
| C₂H₅ | H | H | H | CN | |
| C2H5 | H | H | F | CN | |
| C2H5 | F | F | F | CN | |
| C₂H₅ | F | H | F | CN | |
| H | H | H | H | OCF₃ | |
| H | H | H | F | OCF₃ | |
| H | F | F | F | OCF₃ | |
| H | F | H | F | OCF₃ | |
| H | F | H | H | OCF₃ | K 25 N, 46,3 I; |
| | | | | | Δn = +0,129; Δε = 9,89 |
| CH₃ | H | H | H | OCF₃ | |
| CH₃ | F | H | H | OCF₃ | K 49 N, 91,0 I; |
| | | | | | Δn = +0,140; Δε = 12,39 |
| CH₃ | H | H | F | OCF₃ | |
| CH₃ | F | F | F | OCF₃ | |
| C₂H₅ | H | H | H | OCF₃ | |
| C₂H₅ | H | H | F | OCF₃ | |
| C₂H₅ | F | F | F | OCF₃ | |
| C₂H₅ | F | H | F | OCF₃ | |
| H | H | H | H | CF₃ | |
| H | H | H | F | CF₃ | |
| H | F | F | F | CF₃ | |
| H | F | H | F | CF₃ | |
| R | L¹ | L² | L³ | X | |
| CH₃ | H | H | H | CF₃ | |
| CH₃ | H | H | F | CF₃ | |
| CH₃ | F | F | F | CF₃ | |
| C₂H₅ | H | H | H | CF₃ | |
| C₂H₅ | H | H | F | CF₃ | |
| C₂H₅ | F | F | F | CF₃ | |
| C₂H₅ | F | H | F | CF₃ | |
| H | H | H | H | OCHF₂ | |
| H | H | H | F | OCHF₂ | |
| H | F | F | F | OCHF₂ | K 66 I; Δn = +0,104; |
| | | | | | Δε = 12,23 |
| H | F | H | F | OCHF₂ | |
| CH₃ | H | H | H | OCHF₂ | |
| CH₃ | H | H | F | OCHF₂ | |
| CH₃ | F | F | F | OCHF₂ | |
| C₂H₅ | H | H | H | OCHF₂ | |
| C₂H₅ | H | H | F | OCHF₂ | |
| C₂H₅ | F | F | F | OCHF₂ | |
| C₂H₅ | F | H | F | OCHF₂ | |
| H | H | H | H | OCHFCF₃ | |
| H | H | H | F | OCHFCF₃ | |
| H | F | F | F | OCHFCF₃ | |
| H | F | H | F | OCHFCF₃ | |
| CH₃ | H | H | H | OCHFCF₃ | |
| CH₃ | H | H | F | OCHFCF₃ | |
| CH3 | F | F | F | OCHFCF₃ | |
| C₂H₅ | H | H | H | OCHFCF₃ | |
| C₂H₅ | H | H | F | OCHFCF₃ | |
| C₂H₅ | F | F | F | OCHFCF₃ | |
| C₂H₅ | F | H | F | OCHFCF₃ | |
| H | H | H | H | OC₂F₅ | |
| H | H | H | F | OC₂F₅ | |
| H | F | F | F | OC₂F₅ | |
| H | F | H | F | OC₂F₅ | |
| R | L¹ | L2 | L3 | X | |
| CH₃ | H | H | H | OC₂F₅ | |
| CH₃ | H | H | F | OC₂F₅ | |
| CH₃ | F | F | F | OC₂F₅ | |
| C₂H₅ | H | H | H | OC₂F₅ | |
| C₂H₅ | H | H | F | OC₂F₅ | |
| C₂H₅ | F | F | F | OC₂F₅ | |
| C₂H₅ | F | H | F | OC₂F₅ | |
| H | H | H | H | OC₃F₇ | |
| H | H | H | F | OC₃F₇ | |
| H | F | F | F | OC₃F₇ | |
| H | F | H | F | OC₃F₇ | |
| CH₃ | H | H | H | OC₃F₇ | |
| CH₃ | H | H | F | OC₃F₇ | |
| CH₃ | F | F | F | OC₃F₇ | |
| C₂H₅ | H | H | H | OC₃F₇ | |
| C₂H₅ | H | H | F | OC₃F₇ | |
| C₂H₅ | F | F | F | OC₃F₇ | |
| C₂H₅ | F | H | F | OC₃F₇ | |
| H | H | H | H | C₂F₅ | |
| H | H | H | F | C₂F₅ | |
| H | F | F | F | C₂F₅ | |
| H | F | H | F | C₂F₅ | |
| CH₃ | H | H | H | C₂F₅ | |
| CH₃ | H | H | F | C₂F₅ | |
| CH₃ | F | F | F | C₂F₅ | |
| C₂H₅ | H | H | H | C₂F₅ | |
| C₂H₅ | H | H | F | C₂F₅ | |
| C₂H₅ | F | F | F | C₂F₅ | |
| C₂H₅ | F | H | F | C₂F₅ | |
| H | H | H | H | OCH₂CF₃ | |
| H | H | H | F | OCH₂CF₃ | |
| H | F | F | F | OCH₂CF₃ | |
| H | F | H | F | OCH₂CF₃ | |
| R | L¹ | L² | L3 | X | |
| CH₃ | H | H | H | OCH₂CF₃ | |
| CH₃ | H | H | F | OCH₂CF₃ | |
| CH₃ | F | F | F | OCH₂CF₃ | |
| C₂H₅ | H | H | H | OCH₂CF₃ | |
| C₂H₅ | H | H | F | OCH₂CF₃ | |
| C₂H₅ | F | F | F | OCH₂CF₃ | |
| C₂H₅ | F | H | F | OCH₂CF₃ | |
| H | H | H | H | OCH₂CHF₂ | |
| H | H | H | F | OCH₂CHF₂ | |
| H | F | F | F | OCH₂CHF₂ | K 77 I; Δn = +0,120; Δε = 12,06 |
| H | F | H | F | OCH₂CHF₂ | |
| CH₃ | H | H | H | OCH₂CHF₂ | |
| CH₃ | H | H | F | OCH₂CHF₂ | |
| CH₃ | F | F | F | OCH₂CHF₂ | |
| C₂H₅ | H | H | H | OCH₂CHF₂ | |
| C₂H₅ | H | H | F | OCH₂CHF₂ | |
| C₂H₅ | F | F | F | OCH₂CHF₂ | |
| C₂H₅ | F | H | F | OCH2CHF2 | |

### Mischungsbeispiele

| **Beispiel A** | | |
|---|---|---|
| PCH-5F | 9,50 % | Klärpunkt [°C]: 90,3 |
| PCH-6F | 7,68 % | Δn [589 nm, 20 °C]: +0,0981 |
| PCH-7F | 5,70 % | Δε [1 kHz, 20 °C]: 6,41 |
| CCP-20CF ₃ | 7,60 % | |
| CCP-30CF ₃ | 11,48 % | |
| CCP-40CF ₃ | 8,55 % | |
| CCP-50CF ₃ | 8,55 % | |
| BCH-3F.F | 11,40 % | |
| BCH-5F.F | 9,50 % | |
| ECCP-30CF ₃ | 4,75 % | |
| ECCP-50CF ₃ | 4,75 % | |
| CBC-33F | 1,90 % | |
| CBC-53F | 1,90 % | |
| CBC-55F | 1,90 % | |
| CUG-1V-F | 5,00 % | |

| **Beispiel B** | | |
|---|---|---|
| PCH-5F | 9,0 % | Klärpunkt [°C]: 84 |
| PCH-6F | 7,2 % | Δn [589 nm, 20 °C]: +0,0987 |
| PCH-7F | 5,4 % | Δε [1 kHz, 20 °C]: 6,99 |
| CCP-20CF₃ | 7,2 % | |
| CCP-30CF₃ | 10,8 % | |
| CCP-40CF₃ | 8,1 % | |
| CCP-50CF₃ | 8,1 % | |
| BCH-3F.F | 10,8 % | |
| BCH-5F.F | 9,0 % | |
| ECCP-30CF₃ | 4,5 % | |
| ECCP-50CF₃ | 4,5 % | |
| CBC-33F | 1,8 % | |
| CBC-53F | 1,8 % | |
| CBC-55F | 1,8 % | |
| CUG-V-F | 10,0 % | |

| **Beispiel C** | | |
|---|---|---|
| PCH-5F | 9,0 % | Klärpunkt [°C]: 82,6 |
| PCH-6F | 7,2 % | Δn [589 nm, 20 °C]: +0,0980 |
| PCH-7F | 5,4 % | Δε [1 kHz, 20 °C]: 6,75 |
| CCP-20CF₃ | 7,2 % | |
| CCP-30CF₃ | 10,8 % | |
| CCP-40CF₃ | 8,1 % | |
| CCP-50CF₃ | 8,1 % | |
| BCH-3F.F | 10,8 % | |
| BCH-5F.F | 9,0 % | |
| ECCP-30CF₃ | 4,5 % | |
| ECCP-50CF₃ | 4,5 % | |
| CBC-33F | 1,8 % | |
| CBC-53F | 1,8 % | |
| CBC-55F | 1,8 % | |
| CUU-V-OD | 10,0 % | |

| **Beispiel D** | | |
|---|---|---|
| PCH-5F | 9,0 % | Klärpunkt [°C]: 86,61 |
| PCH-6F | 7,2 % | Δn [589 nm, 20 °C]: +0,0996 |
| PCH-7F | 5,4 % | Δε [1 kHz, 20 °C]: 6,51 |
| CCP-20CF₃ | 7,2 % | |
| CCP-30CF₃ | 10,8 % | |
| CCP-40CF₃ | 8,1 % | |
| CCP-50CF₃ | 8,1 % | |
| BCH-3F.F | 10,8 % | |
| BCH-5F.F | 9,0 % | |
| ECCP-30CF₃ | 4,5 % | |
| ECCP-50CF₃ | 4,5 % | |
| CBC-33F | 1,8 % | |
| CBC-53F | 1,8 % | |
| CBC-55F | 1,8 % | |
| CUU-V-O1D | 10,0 % | |

| **Beispiel E** | | |
|---|---|---|
| PCH-5F | 3,60 % | Klärpunkt [°C]: 116 |
| CCP-20CF₂ .F.F | 19,17 % | Δε [589 nm, 20 °C]: 9,1 |
| CCP-30CF₂ .F.F | 18,00 % | K ₃ /K ₁ : 1,55 |
| CCP-50CF₂ .F.F | 19,17 % | V _{(10,0,20)} [V]: 1,16 |
| CUP-2F.F | 6,03 % | |
| CUP-3F.F | 6,03 % | |
| CBC-33F | 6,03 % | |
| CBC-53F | 6,03 % | |
| CBC-55F | 5,94 % | |
| CGU-1V-F | 10,00 % | |

| **Beispiel F** | | |
|---|---|---|
| PCH-5F | 9,0 % | Klärpunkt [°C]: 88,3 |
| PCH-6F | 7,2 % | Δn [589 nm, 20 °C]: 0,1008 |
| PCH-7F | 5,4 % | Δε [1 kHz, 20 °C]: 5,88 |
| CCP-20CF₃ | 7,2 % | Viskosität [20 °C]: 15 |
| CCP-30CF₃ | 10,8 % | |
| CCP-40CF₃ | 8,1 % | |
| CCP-50CF₃ | 8,1 % | |
| BCH-3F.F | 10,8 % | |
| BCH-5F.F | 9,0 % | |
| ECCP-30CF₃ | 4,5 % | |
| ECCP-50CF₃ | 4,5 % | |
| CBC-33F | 1,8 % | |
| CBC-53F | 1,8 % | |
| CBC-55F | 1,8 % | |
| CGG-V-F | 10,0 % | |

## Patentansprüche

1. Flüssigkristallines Medium enthaltend ein oder mehrere fluorierte Biphenylcyclohexane der Formel I, 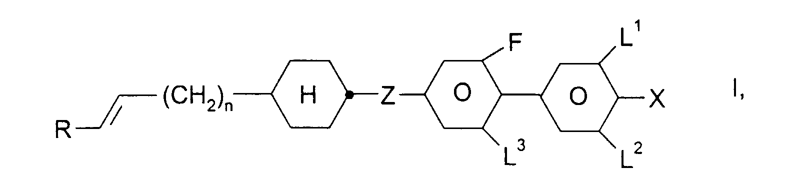 worin
R H, Alkyl-, Oxaalkyl- oder Oxaalkenylrest mit 1-6 C-Atomen,
X F, Cl, CN, ein durch ein oder mehrere Fluoratome substituierter Alkyl-, Alkoxy-, Alkenyloxy oder Alkenylrest mit 1 bis 6 C-Atomen,
L¹, L² und L³ jeweils unabhängig voneinander H oder F,
Z -CH₂CH₂-, -CH=CH-, -C≡C- oder eine Einfachbindung, und
n 0, 1, 2 oder 3,
bedeuten,
und ein oder mehrere Verbindungen ausgewählt aus der Gruppe bestehend aus den allgemeinen Formeln II, III und IV, 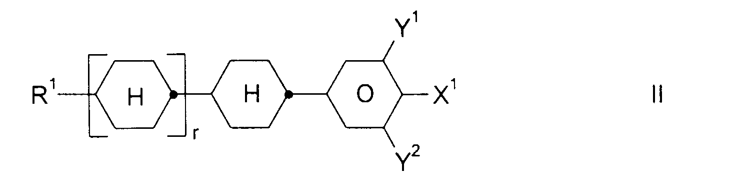 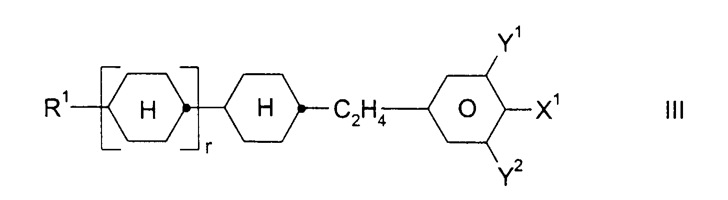 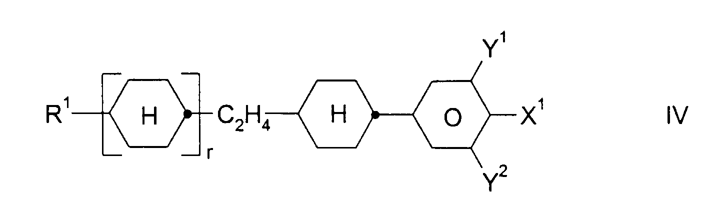 worin
R¹: Alkyl, Oxaalkyl, Fluoralkyl oder Alkenyl mit jeweils bis zu 7 C-Atomen
X¹: F, Cl, CF₃, OCF₃ oder OCHF₂
Y¹ und Y²: jeweils unabhängig voneinander H oder F und
r: 0 oder 1
bedeuten,
wobei der Anteil an Verbindungen der Formeln I bis IV im Gesamtgemisch mindestens 50 Gew.-% beträgt.

2. Flüssigkristallines Medium nach Anspruch 1, dadurch gekennzeichnet, daß X in Formel I F, CN, CF₃, OCF₃, OCHF₂, OCHFCF₃, OCH₂CF₃, OCH₂CHF₂, OC₂F₅, OC₃F₇ oder C₂F₅ ist.

3. Flüssigkristallines Medium nach Anspruch 1 oder 2, dadurch gekennzeichnet, daß R in Formel I H oder CH₃ ist.

4. Flüssigkristallines Medium nach einem der Ansprüche 1 bis 3, dadurch gekennzeichnet, daß es ein Biphenylcyclohexan der Formel I2, 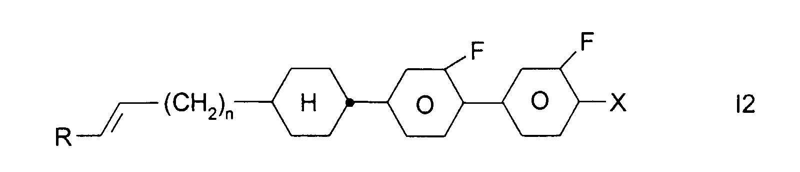 worin
R, X und n die in Anspruch 1 angegebene Bedeutung haben,
enthält.

5. Flüssigkristallines Medium nach einem der Ansprüche 1 bis 3, dadurch gekennzeichnet, daß es ein Biphenylcyclohexan der Formel I5, 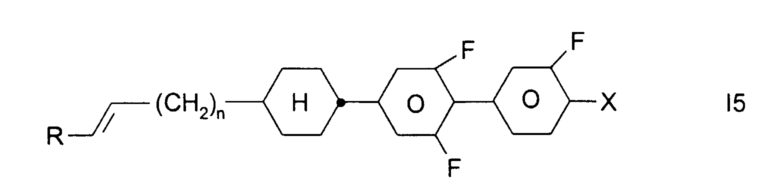 worin
R, X und n die in Anspruch 1 angegebene Bedeutung haben,
enthält.

6. Flüssigkristallines Medium nach einem der Ansprüche 1 bis 3, dadurch gekennzeichnet, daß es ein Biphenylcyclohexan der Formel I6, 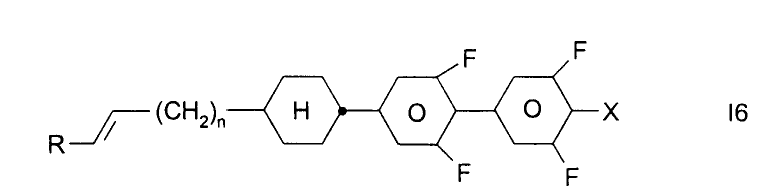 worin
R, X und n die in Anspruch 1 angegebene Bedeutung haben,
enthält.

7. Flüssigkristallines Medium nach einem der Ansprüche 1 bis 6, dadurch gekennzeichnet, daß es ein oder mehrere Verbindungen der Formel I3, 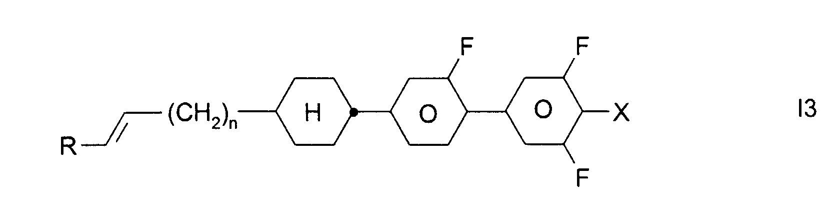 worin
R, X und n die in Anspruch 1 angegebenen Bedeutungen haben,
enthält.

8. Flüssigkristallines Medium nach einen der Ansprüche 1 bis 7, dadurch gekennzeichnet, daß es ein oder mehrere Verbindungen der Formel 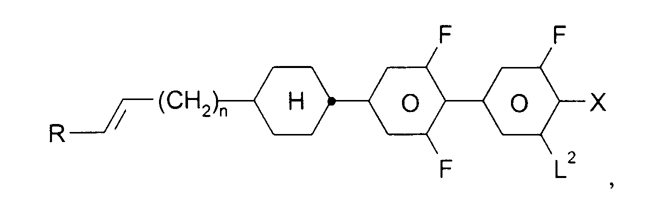 worin
R, X, L und n die in Anspruch 1 angegebenen Bedeutungen haben,
enthält.

9. Flüssigkristallines Medium nach einem der Ansprüche 1 bis 8, dadurch gekennzeichnet, daß X F, Cl, CN, CF₃, OCHF₂, OCF₃ oder OCHFCF₃ ist.

10. Verwendung des flüssigkristallinen Mediums nach Anspruch 1 für elektrooptische Zwecke.

11. Elektrooptische Flüssigkristallanzeige enthaltend ein flüssigkristallines Medium nach Anspruch 1.

## Claims

1. Liquid-crystalline medium comprising one or more fluorinated biphenylcyclohexanes of the formula I 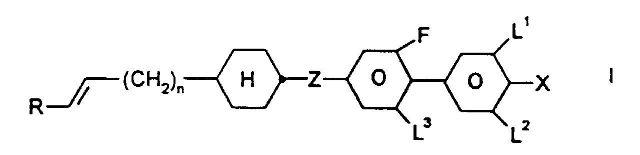 in which
R is H or an alkyl, oxaalkyl or oxaalkenyl radical having 1-6 carbon atoms,
X is F, Cl, CN, or an alkyl, alkoxy, alkenyloxy or alkenyl radical having 1 to 6 carbon atoms which is substituted by one or more fluorine atoms,
L¹ , L² and L³ are each, independently of one another, H or F,
Z is -CH₂CH₂-, -CH=CH-, -C≡C- or a single bond, and
n is 0, 1, 2 or 3,
and one or more compounds selected from the group consisting of the general formulae II, III and IV, 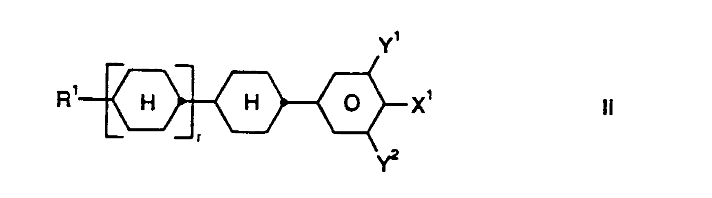 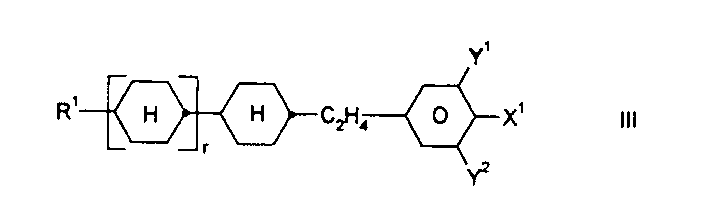 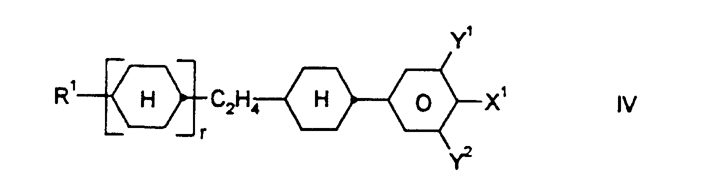 in which
R¹: is alkyl, oxaalkyl, fluoroalkyl or alkenyl having, in each case, up to 7 carbon atoms
X¹: is F, Cl, CF₃, OCF₃ or OCHF₂
Y¹ and Y² : are each, independently of one another, H or F and
r: is 0 or 1,
where the proportion of compounds of the formulae I to IV in the total mixture is at least 50% by weight.

2. Liquid-crystalline medium according to Claim 1, characterized in that X in formula I is F, CN, CF₃, OCF₃, OCHF₂, OCHFCF₃, OCH₂CF₃, OCH₂CHF₂, OC₂F₅, OC₃F₇ or C₂F₅.

3. Liquid-crystalline medium according to Claim 1 or 2, characterized in that R in formula I is H or CH₃.

4. Liquid-crystalline medium according to one of Claims 1 to 3, characterized in that it comprises a biphenylcyclohexane of the formula I2 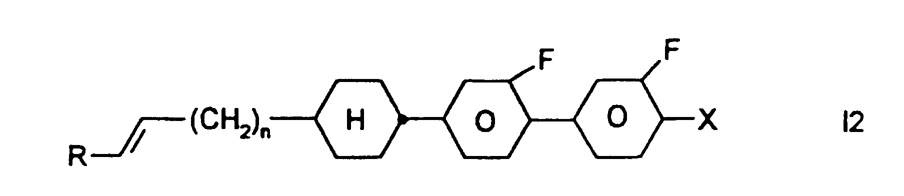 in which
R, X and n are as defined in Claim 1.

5. Liquid-crystalline medium according to one of Claims 1 to 3, characterized in that it comprises a biphenylcyclohexane of the formula I5 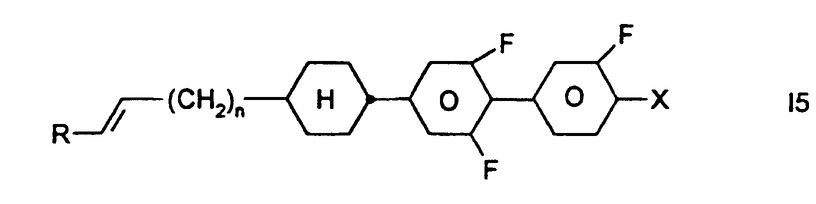 in which
R, X and n are as defined in Claim 1.

6. Liquid-crystalline medium according to one of Claims 1 to 3, characterized in that it comprises a biphenylcyclohexane of the formula I6 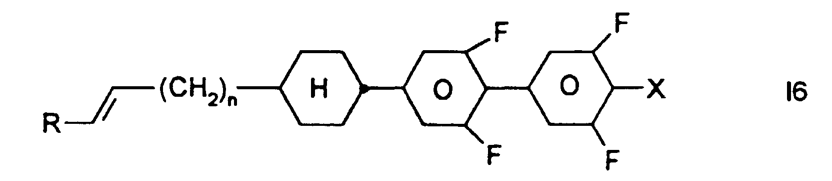 in which
R, X and n are as defined in Claim 1.

7. Liquid-crystalline medium according to one of Claims 1 to 6, characterized in that it comprises one or more compounds of the formula I3 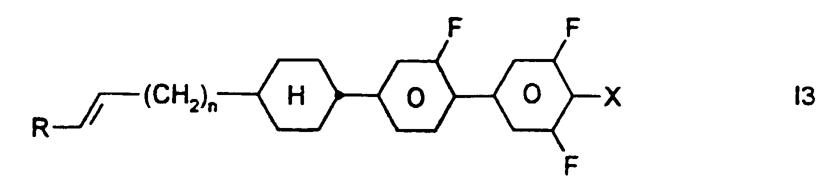 in which R, X and n are as defined in Claim 1.

8. Liquid-crystalline medium according to Claim 1 to 7, characterized in that it comprises one or more compounds of the formula 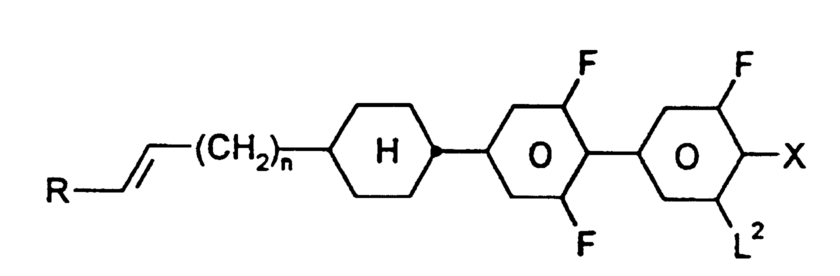 in which
R, X, L and n are as defined in Claim 1.

9. Liquid-crystalline medium according to one of Claims 1 to 8, characterized in that X is F, Cl, CN, CF₃, OCHF₂, OCF₃ or OCHFCF₃.

10. Use of the liquid-crystalline medium according to Claim 1 for elctro-optical purposes.

11. Electro-optical liquid-crystal display comprising a liquid-crystalline medium according to Claim 1.

## Revendications

1. Milieu à cristaux liquides contenant un ou plusieurs biphénylcyclohexanes fluorés de formule I : 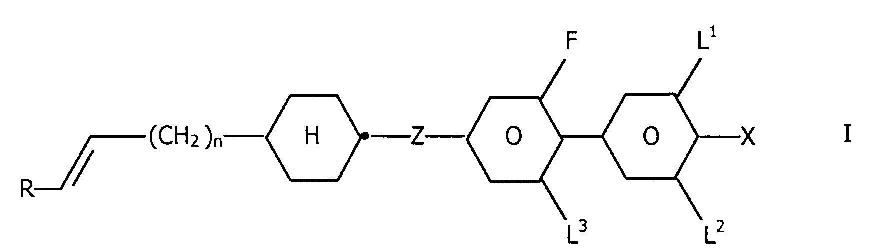 dans laquelle
R représente l'hydrogène, un groupe alkyle, oxa-alkyle ou oxa-alcényle en C₁-C₆,
X représente F, Cl, un groupe CN, un groupe alkyle, alcoxy, alcényloxy ou alcényle en C₁-C₆ substitué par un ou plusieurs atomes de fluor,
L¹, L² et L³ représentent chacun, indépendamment les uns des autres, H ou F,
Z représente -CH₂CH₂-, -CH=CH-, -C≡C- ou une liaison simple et
n est égal à 0, 1, 2 ou 3
et un ou plusieurs composés choisis dans le groupe consistant en les composés de formules générales II, III et IV : 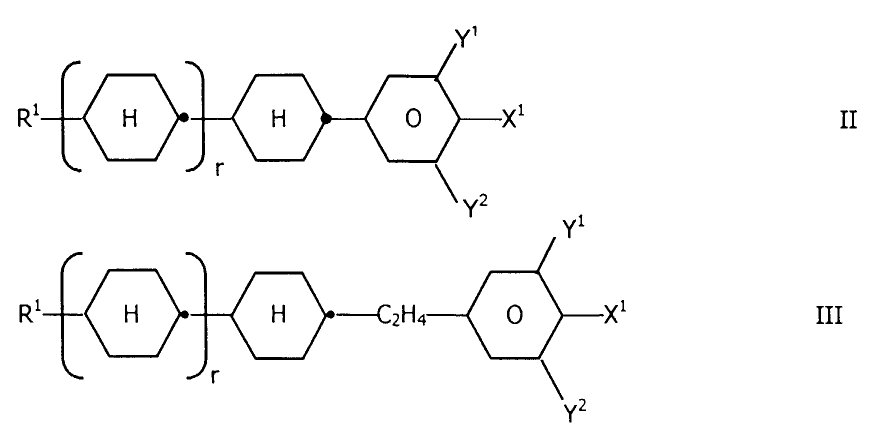 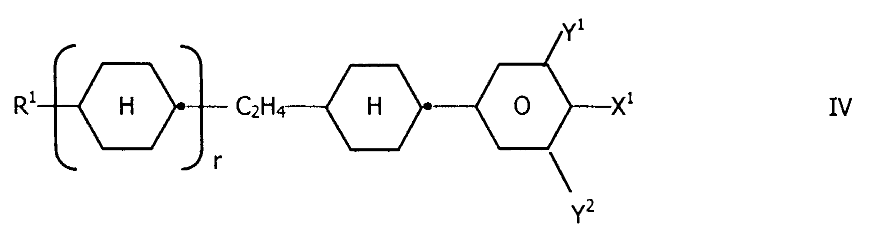 dans lesquelles
R¹ représente un groupe alkyle, oxa-alkyle, fluoralkyle ou alcényle contenant chacun jusqu'à 7 atomes de carbone,
X¹ représente F, Cl, CF₃, OCF₃ ou OCHF₂,
Y¹ et Y² représentent chacun, indépendamment l'un de l'autre, H ou F et
r est égal à 0 ou 1,
la proportion des composés de formules I à IV représentant au moins 50 % du poids du mélange total.

2. Milieu à cristaux liquides selon la revendication 1, caractérisé en ce que, dans la formule I, X représente F, CN, CF₃, OCF₃, OCHF₂, OCHFCF₃, OCH₂CF₃, OCH₂CHF₂, OC₂F₅, OC₃F₇ ou C₂F₅.

3. Milieu à cristaux liquides selon la revendication 1 ou 2, caractérisé en ce que, dans la formule I, R représente H ou CH₃.

4. Milieu à cristaux liquides selon l'une des revendications 1 à 3, caractérisé en ce qu'il contient un biphénylcyclohexane de formule I2 : 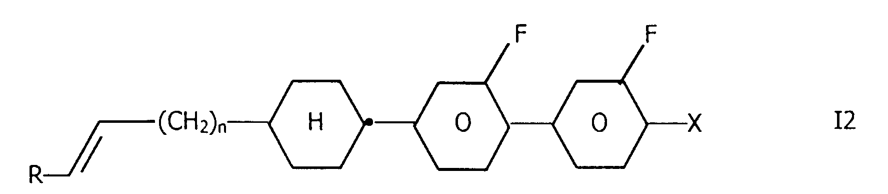 dans laquelle :
R, X et n ont les significations indiquées dans la revendication 1.

5. Milieu à cristaux liquides selon l'une des revendications 1 à 3, caractérisé en ce qu'il contient un biphénylcyclohexane de formule I5 : 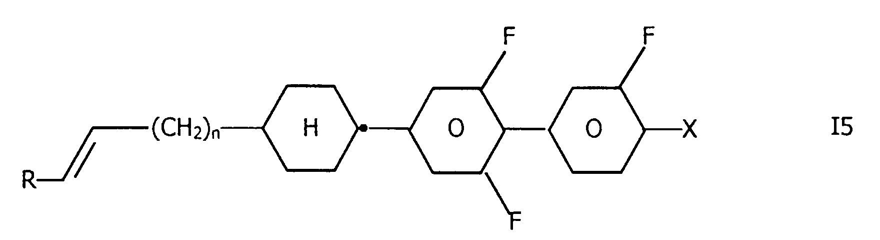 dans laquelle
R, X et n ont les significations indiquées dans la revendication 1.

6. Milieu à cristaux liquides selon l'une des revendications 1 à 3, caractérisé en ce qu'il contient un biphénylcyclohexane de formule I6 : 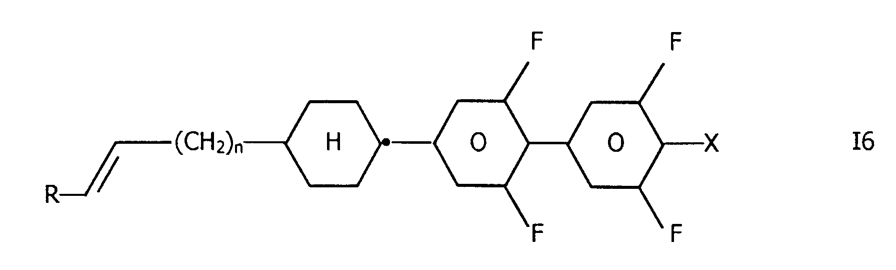 dans laquelle
R, X et n ont les significations indiquées dans la revendication 1.

7. Milieu à cristaux liquides selon l'une des revendications 1 à 6, caractérisé en ce qu'il contient un ou plusieurs composés de formule I3 : 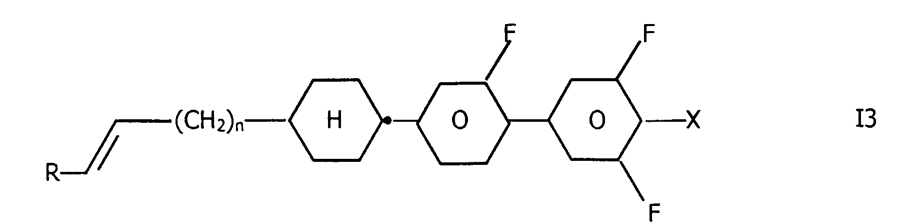 dans laquelle
R, X et n ont les significations indiquées dans la revendication 1.

8. Milieu à cristaux liquides selon l'une des revendications 1 à 7, caractérisé en ce qu'il contient un ou plusieurs composés de formule : 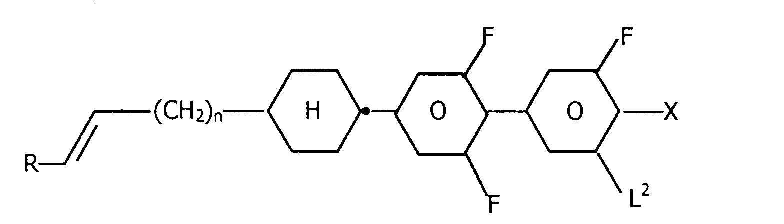 dans laquelle
R, X, L² et n ont les significations indiquées dans la revendication 1.

9. Milieu à cristaux liquides selon l'une des revendications 1 à 8, caractérisé en ce que X représente F, Cl, CN, CF₃, OCHF₂, OCF₃ ou OCHFCF₃.

10. Utilisation du milieu à cristaux liquides selon la revendication 1 pour des applications électro-optiques.

11. Dispositif d'affichage électro-optiques à cristaux liquides contenant un milieu à cristaux liquides selon la revendication 1.
